# EUROPEAN PATENT SPECIFICATION

(11) **EP 3 870 272 B1**
(45) Date of publication and mention of the grant of the patent: **02.07.2025**
(21) Application number: 19874940.0
(22) Date of filing: 23.10.2019
(51) Int. Cl.: A61N 1/36, A61N 1/04, A61B 5/369, A61B 5/0205, A61B 5/00, A61N 1/05, A61N 1/372, A61N 2/00, A61N 7/00

(54) **NERVE STIMULATION FOR TREATING MIGRAINE AND OTHER HEADACHE CONDITIONS**
NERVENSTIMULATION ZUR BEHANDLUNG VON MIGRÄNE UND ANDEREN KOPFSCHMERZEN
STIMULATION NERVEUSE POUR LE TRAITEMENT DE LA MIGRAINE ET D'AUTRES ÉTATS DE CÉPHALÉE

(30) Priority: 24.10.2018 US 201862750204 P
(43) Date of publication of application: 01.09.2021
(60) Divisional application: 25178596.0
(73) Proprietor: Cala Health, Inc., San Mateo, CA 94404 (US)
(72) Inventor: HAMNER, Samuel, Richard, Burlingame, California 94010 (US); KENT, Alexander, R, Burlingame, California 94010 (US); ROSENBLUTH, Kathryn H., Burlingame, California 94010 (US)
(74) Representative: J A Kemp LLP
(86) International application number: PCT/US2019/057674
(87) International publication number: WO 2020/086726

(56) References cited:
- WO-A1-2017/208167
- WO-A1-2018/039458
- WO-A1-2018/093765
- WO-A1-2019/143790
- WO-A1-2020/006048
- US-A1- 2004 167 588
- US-A1- 2011 230 701
- US-A1- 2012 078 319
- US-A1- 2015 321 000
- US-A1- 2017 056 238

## Description

### BACKGROUND

### Field of the Invention

Embodiments of the invention relate generally to the treatment of migraine, and optionally including cephalgia and headache, and more specifically to systems of treating migraine, and optionally cephalgia, headache, and other headache conditions through noninvasive peripheral nerve stimulation.

### Description of the Related Art

WO 2018/039458, WO 2018/093765 and US 2015/321000 describe prior art stimulation methods and apparatuses. WO 2018/093765 discloses a wearable device for treating migraines using transcutaneous peripheral nerve stimulation, comprising: a peripheral nerve effector configured to be placed on a skin surface proximate a nerve of a patient; and a controller configured to deliver electrical stimulation signal to the patient via the peripheral nerve effector.

Migraine is a brain disorder of dysregulation of sensory processing associated with autonomic involvement. Migraine is a neurovascular disorder with symptoms including unilateral throbbing cranial pain, sensory sensitivity to light, sound, and smell, nausea, and dysfunction of autonomic, cognitive, emotional, and motor systems. Migraines can be triggered both internally or externally, including factors like stress, hormonal changes, sleep deprivation, malnourishment, or sensory overload. In about one-third of cases, symptoms, such as auras, occur prior to the onset of a migraine episode. Up to 16% of the world population suffer from migraines, and they are most common in women, and have a strong genetic component.

Migraines impact over 39 million Americans, making it the second leading cause of neurological disability. Current treatments are often ineffective or poorly tolerated - preventative pharmacologic medications are effective for only about 50% of patients, and long term effectiveness is poor with benefit only for 25% at 6 months and 14% at 12 months according to some studies.

The onset of migraines is multifactorial and an episode is thought to be triggered by activation of nociceptors innervating vascular structures of the brain by mechanical, electrical, and/or chemical triggers. The nociceptors innervating the vascular structures consist of C-fibers and A-fibers that contain neuropeptides such as substance P and calcitonin gene-related peptide (CGRP). The nociceptors originate in the trigeminal ganglion and reach the intracranial structures through the trigeminal nerve.

The hypothalamus is deeply involved in a migraine attack, including increasing susceptibility, attack triggering, and symptomology. The orexins (alternatively called hypocretins) are a pair of hypothalamic neuropeptides synthesized in cell bodies exclusively in the lateral hypothalamus. Anatomically, the orexin system is closely integrated with other brain systems involved in migraine pathophysiology, including the trigeminovascular system. Orexinergic systems can contribute to altered homeostatic mechanisms that can influence attack susceptibility, premonitory and associated symptoms, and migraine nociception; as such, this system can be a potential therapeutic target for migraine.

### SUMMARY

The present invention relates generally to the treatment of migraine, and optionally cephalgia, headache, and other cranial pain, and more specifically to systems and devices for treating migraine, and optionally cephalgia, headache, and other cranial pain through noninvasive peripheral nerve stimulation.

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes.

In some embodiments, disclosed herein is a system for treating migraine in a patient. The system can include, or consist essentially of a peripheral nerve stimulator including a pulse generator and at least two electrodes configured to deliver electrical neuromodulation, e.g., stimulation to a nerve, acupressure point, or meridian in the patient's limb or ear. As disclosed herein, a patient's ear is discrete and separate anatomically from the patient's head. In some embodiments, the stimulation is not delivered to the patient's head, but rather on the patient's ear, lower extremity, or upper extremity. The upper extremity neuromodulation, e.g., stimulation could be in some embodiments on the upper arm, spanning the upper arm and lower arm, or only below the elbow (e.g., only on one or more of the forearm, wrist, and/or finger(s)), and the stimulation devices can be configured accordingly. The neuromodulation, e.g., stimulation can be sufficient in some embodiments to reduce one or more of throbbing cranial pain, sensory sensitivity to light, sound, and smell, nausea, and dysfunction of autonomic, cognitive, emotional, and motor systems. In some embodiments, the neuromodulation, e.g., stimulation could be therapeutic (e.g., after the start of symptoms), and/or prophylactic/preventative (e.g., when the patient is not currently experiencing any symptoms). The system can also include one, two, or more sensors. The stimulator and/or the sensor could be implantable within a patient, or wearable without any implantable components. The stimulator and/or the sensor(s) could be percutaneous or transcutaneous in some embodiments.

As used herein, neuromodulation can include both excitatory (e.g., stimulation) as well as inhibitory (e.g., block) therapy to one or more nerves.

In some embodiments, systems and methods for treatment of migraine (the methods being useful for understanding the invention) can include, or consist essentially of any number of features as disclosed herein in the specification.

Disclosed herein is a transcutaneous method for treating migraine with selective activation, the method is useful for understanding the invention. The method can include, for example, or consist essentially of positioning a first peripheral nerve effector on a patient's skin on an extremity of the patient; delivering a first electrical nerve stimulation signal transcutaneously to the first peripheral nerve effector to stimulate a first peripheral nerve sufficient to modify at least one neural pathway associated with migraine or headache conditions, including but not limited to the orexinergic pathway and/or the trigeminovascular pathway. Episodic migraineurs can have orexin measurable in the cerebrospinal fluid (CSF). The first electrical nerve stimulation signal can preferentially, or selectively only activate on or more of A-alpha, A-beta, A-delta, B, or C-fibers of the first peripheral nerve. The first peripheral nerve could be an upper extremity nerve, such as, for example, the median nerve, the radial nerve, the medial cutaneous nerve, the lateral cutaneous nerve, the musculocutaneous nerve, or the ulnar nerve; a cranial or cervical nerve, such as the auricular nerves, specifically the auricular branch of the vagus nerve (ABVN), the vagus nerve, specifically the cervical vagus nerve, the trigeminal nerve, specifically the supraorbital nerve, the occipital nerve, or the sphenopalatine ganglion; or a lower extremity nerve such as, for example, the tibial nerve, the saphenous nerve, the common peroneal nerve, the femoral nerve, the sacral nerve, the sciatic nerve, and the sural nerve. In some embodiments, the first peripheral nerve does not include any one or more of the foregoing nerves, or any one or more nerves described elsewhere herein. The first electrical nerve stimulation signal could include burst or continuous stimulation, and of a selected waveform that could a biphasic square waveform or sinusoidal in some cases. The pulse width could be, for example, between about 50 µs and about 100µs, between about 150µs and about 200µs, between about 300µs and about 400µs, or other ranges included any two of the aforementioned values. In some embodiments, the electrical stimulation signal could have a frequency of about 5-50 Hz,, 50-150 Hz, 150-250 Hz, 250-500 Hz, 500-1000 Hz, 1000-2500 Hz, and overlapping ranges therein, and including for example about, no more than about, or at least about 5 Hz, about 250 Hz, or about 2,000 Hz. In some embodiments, the first peripheral nerve effector can include at least first and second electrodes. The electrodes can be substantially aligned along the length of the nerve axon in some cases. The method can include positioning a second peripheral nerve effector on a patient's skin on the extremity of the patient; and delivering a second electrical nerve stimulation signal transcutaneously to the second peripheral nerve effector to stimulate a second peripheral nerve sufficient to modify at least one neural pathway associated with migraine or headache conditions. In some embodiments, the headache condition could be one or more of a sinus headache, cluster headache, tension headache, rebound headache, daily persistent headache, cervicogenic headache, chronic daily headache, occipital neuralgia, hemicranias continua, and the like. In some embodiments, the system stimulates one or more peripheral nerves to balance parasympathetic or sympathetic nervous system activity of the patient. The second peripheral nerve could be different from the first peripheral nerve, and selected from, for example, any of the nerves described elsewhere herein, and/or does not include any of the nerves described elsewhere herein. The method can also include receiving an input relating to autonomic nervous system activity of the patient, including, for example, receiving data from a sensor that measures heart rate variability of the patient; and/or receiving data from a sensor that measures at least one of electrodermal activity, thermometry, and ECG information of the patient. The method can also include positioning any number of the peripheral nerve effectors over one or more of: the C5, C6, C7, C8, T1, T2, T3, T4, T5, T6, T7, T8, T9, T10, T11, T12, L1, L2, L3, L4, L5, S1, S2, S3, and/or S4 dermatomes. In some embodiments, a peripheral nerve effector can be positioned on an extremity of the patient offset from one or more nerves, such as the median nerve, radial nerve, or ulnar nerve for example, and targeting a target nerve, such as a cutaneous nerve. In some embodiments, an electrical nerve stimulation signal can preferentially activate, or selectively activate only one of A-alpha, A-beta, A-delta, B, or C-fibers of the first peripheral nerve.

Also disclosed herein in some embodiments is a wearable transcutaneous system for treating migraine and optionally other headache conditions with selective activation. The system can include, or consist essentially of any number of the following features, or others disclosed elsewhere in the specification. The system can include, for example, a controller; a first peripheral nerve effector configured to be positioned on a patient's skin on an extremity of the patient; and/or at least one biomedical sensor or data input source configured to provide feedback information. The controller can be configured to generate a first electrical nerve stimulation signal transcutaneously to the first peripheral nerve effector to stimulate a first peripheral nerve sufficient to modify at least one neural pathway associated with migraine or headache conditions. The first electrical nerve stimulation signal can preferentially or selectively activate one or more of A-alpha, A-beta, A-delta, or C-fibers of the first peripheral nerve. The system can also include a second peripheral nerve effector configured to be positioned on the patient's skin on the extremity of the patient. The controller can be configured to generate a second electrical nerve stimulation signal transcutaneously to the second peripheral nerve effector to stimulate a second peripheral nerve sufficient to modify at least one neural pathway associated with migraine or headache conditions. The second electrical nerve stimulation signal can preferentially activate one or more of A-alpha, A-beta, A-delta, B, or C-fibers of the second peripheral nerve. The feedback information can include, for example, heart rate variability and/or galvanic skin response. The first peripheral nerve could be an upper extremity nerve, such as, for example, the median nerve, the radial nerve, the medial cutaneous nerve, the lateral cutaneous nerve, the musculocutaneous nerve, or the ulnar nerve; a cranial or cervical nerve, such as the auricular nerves, specifically the ABVN, the vagus nerve, specifically the cervical vagus nerve, the trigeminal nerve, specifically the supraorbital nerve, the occipital nerve, or the sphenopalatine ganglion; or a lower extremity nerve such as, for example, the tibial nerve, the saphenous nerve, the common peroneal nerve, the femoral nerve, the sacral nerve, the sciatic nerve, and the sural nerve. The first electrical nerve stimulation signal could include burst or continuous stimulation, and of a selected waveform that could a biphasic square waveform or sinusoidal in some cases. The pulse width could be, for example, between about 50 µs and about 100µs, between about 150µs and about 200µs, between about 300µs and about 400µs, or other ranges included any two of the aforementioned values. In some embodiments, the electrical stimulation signal could have a frequency of about 5-50 Hz, 50-100Hz, 100-250Hz, 250-500Hz, 500Hz-1000Hz, 1000-3000Hz, and ranges in between, including for example about, at least about, or no more than about 5 Hz, about 250 Hz, or about 2,000 Hz. In some embodiments, the first peripheral nerve effector can include at least first and second electrodes. The electrodes can be substantially aligned along the length of the nerve axon in some cases. In some embodiments, the system can include a second peripheral nerve effector configured to be positioned on a patient's skin on the extremity of the patient; and configured to deliver a second electrical nerve stimulation signal transcutaneously to the second peripheral nerve effector to stimulate a second peripheral nerve sufficient to modify at least one brain or spinal cord autonomic feedback loop and balance parasympathetic or sympathetic nervous system activity of the patient. The second peripheral nerve could be different from the first peripheral nerve, and selected from, for example, any of the nerves described elsewhere herein. The system can also be configured to receive an input relating to autonomic nervous system activity of the patient, including, for example, receiving data from a sensor that measures heart rate variability of the patient; and/or receiving data from a sensor that measures at least one of electrodermal activity, thermometry, and ECG information of the patient.

Useful for understanding the invention, disclosed herein are methods for treating migraine and headache conditions, that can include one or more of, or consist essentially of positioning a first peripheral nerve effector on a patient's skin on an upper extremity of the patient to stimulate a first peripheral nerve selected from the group consisting of one of a median nerve, radial nerve, and ulnar nerve of the patient; positioning a second peripheral nerve effector on the patient's skin on the upper extremity of the patient to stimulate a second peripheral nerve different from the first peripheral nerve; delivering a first electrical nerve stimulation signal transcutaneously to the first peripheral nerve effector to stimulate the first peripheral nerve sufficient to modify at least one neural pathway associated with migraine or headache conditions; and/or delivering a second electrical nerve stimulation signal transcutaneously to the second peripheral nerve effector to stimulate the second peripheral nerve sufficient to modify at least one neural pathway associated with migraine or headache conditions. The first electrical nerve stimulation signal and the second electrical nerve stimulation signal can be coordinated such that stimulation from the first peripheral nerve effector and stimulation from the second peripheral nerve effector activate the brachial plexus concurrently. The second electrical nerve stimulation signal can occur simultaneously or substantially simultaneously with delivering the first electrical nerve stimulation signal. Delivering the second electrical nerve stimulation signal can be offset temporally from delivering the first electrical nerve stimulation signal, such as between about 0.5-10 milliseconds (e.g., about 1-3 milliseconds, 1.0 millisecond to about 2.1 milliseconds in some cases, and overlapping ranges therein). The method can also include performing a nerve conduction study to measure a nerve conduction velocity of the first peripheral nerve and the second peripheral nerve. The offset can be determined from the measured nerve conduction velocity of the first peripheral nerve and the second peripheral nerve. The nerve stimulation signals can be delivered in alternating and/or rhythmic patterns, such as frequencies (e.g., an alternating frequency) of between about 3 Hz and about 15 Hz. Frequencies of between about 1-20 Hz may be used in some embodiments. The nerve stimulation signals can be delivered in a pseudorandom pattern, and/or be adjusted based on feedback received regarding the autonomic balance of the patient. The feedback can include, for example, measured heart rate variability of the patient, such as a ratio of absolute low frequency to absolute high frequency of heart rate variability of the patient. The first peripheral nerve effector and the second peripheral nerve effector span a plurality of dermatomes on the patient, such as any of the dermatomes mentioned herein. The dermatomes can be stimulated at a pre-determined interval.

Also disclosed herein in some embodiments are wearable systems for treating migraine and optionally other headache conditions. The system can include, or consist essentially of any number of the following features, or others disclosed elsewhere in the specification. The systems can include any number of a controller; a first peripheral nerve effector configured to be positioned on a patient's skin on an extremity of the patient; a second peripheral nerve effector configured to be positioned on the patient's skin on the extremity of the patient; and at least one biomedical sensor or data input source configured to provide feedback information. The controller can be configured to generate a first electrical nerve stimulation signal transcutaneously to the first peripheral nerve effector to stimulate a first peripheral nerve sufficient to modify at least one brain or spinal cord autonomic feedback loop relating to the migraine episode or headache condition. The controller can also be configured to generate a second electrical nerve stimulation signal transcutaneously to the second peripheral nerve effector to stimulate a second peripheral nerve sufficient to modify at least one neural pathway associated with migraine or headache conditions. The controller can also be configured to coordinate the first electrical nerve stimulation signal and the second electrical nerve stimulation signal such that stimulation from the first peripheral nerve effector and stimulation from the second peripheral nerve effector activate the brachial plexus concurrently, such as simultaneously or substantially simultaneously with delivering the first electrical nerve stimulation signal. The controller can be configured to deliver the second electrical nerve stimulation signal offset temporally from delivering the first electrical nerve stimulation signal, such as between about 1.0 and about 2.1 milliseconds, 0.5-10 milliseconds (e.g., about 1-3 milliseconds. The controller can be configured to deliver electrical nerve stimulation signals in alternating, random, pseudorandom, and/or rhythmic patterns, such as at frequencies (e.g., an alternating frequency) of between about 3 Hz and about 15 Hz. Frequencies of between about 1-20 Hz may be used in some embodiments. The controller can be configured to adjust at least one of the first electrical stimulation signal and the second electrical nerve stimulation signal based on feedback received regarding the autonomic balance of the patient. The feedback can be, for example, measured heart rate variability of the patient, e.g., a ratio of absolute low frequency to absolute high frequency of heart rate variability of the patient. The controller can be configured to receive recorded measurements regarding the nerve conduction velocity of the first peripheral nerve and the second peripheral nerve, and coordinate the first electrical nerve stimulation signal and the second electrical nerve stimulation signal based upon the recorded measurements.

In some embodiments, a fully or partially transcutaneous system for treating migraine and optionally other neurological condition is provided. The technology may be partially transcutaneous and partially subcutaneous. In some embodiments, the system comprises a first nerve effector that delivers a first neuromodulation signal to modify at least one neural pathway associated with the condition, including but not limited to the orexinergic pathway and/or the trigeminovascular pathway. The first neuromodulation signal can preferentially, or selectively only activate on or more of A-alpha, A-beta, A-delta, B, or C-fibers of the first peripheral nerve. The first peripheral nerve could be an upper extremity nerve, such as, for example, the median nerve, the radial nerve, the medial cutaneous nerve, the lateral cutaneous nerve, the musculocutaneous nerve, or the ulnar nerve; a cranial or cervical nerve, such as the auricular nerves, specifically the auricular branch of the vagus nerve (ABVN), the vagus nerve, specifically the cervical vagus nerve, the trigeminal nerve, specifically for example the supraorbital nerve, the occipital nerve, or the sphenopalatine ganglion; or a lower extremity nerve such as, for example, the tibial nerve, the saphenous nerve, the common peroneal nerve, the femoral nerve, the sacral nerve, the sciatic nerve, and the sural nerve. The first neuromodulation signal could include burst or continuous stimulation, and of a selected waveform that could a biphasic square waveform or sinusoidal in some cases. The pulse width could be, for example, between about 50 µs and about 100µs, between about 150µs and about 200µs, between about 300µs and about 400µs, or other ranges included any two of the aforementioned values. In some embodiments, the electrical stimulation signal could have a frequency of about 5-50 Hz,, 50-150 Hz, 150-250 Hz, 250-500 Hz, 500-1000 Hz, 1000-2500 Hz, and overlapping ranges therein, and including for example about 5 Hz, about 250 Hz, or about 2,000 Hz. In some embodiments, the first nerve effector can include at least first and second electrodes. The electrodes can be substantially aligned along the length of the nerve axon in some cases. In some embodiments, the system can include delivering a second neuromodulation signal to stimulate a second nerve sufficient to modify at least one neural pathway. In some embodiments, the system modulates one or more nerves to balance parasympathetic or sympathetic nervous system activity of the patient. The system and method can also include receiving an input relating to autonomic nervous system activity of the patient, including, for example, receiving data from a sensor that measures heart rate variability of the patient; and/or receiving data from a sensor that measures at least one of electrodermal activity, thermometry, and ECG information of the patient. The system and method can also include positioning any number of the peripheral nerve effectors over one or more of: the C5, C6, C7, C8, T1, T2, T3, T4, T5, T6, T7, T8, T9, T10, T11, T12, L1, L2, L3, L4, L5, S1, S2, S3, and/or S4 dermatomes. In some embodiments, a nerve effector can be positioned on an extremity of the patient offset from one or more nerves, such as the median nerve, radial nerve, or ulnar nerve for example, and targeting a target nerve, such as a cutaneous nerve. In some embodiments, a neuromodulation signal can preferentially affect (activate, inhibit, etc.), or selectively affect (activate, inhibit, etc.) only one of A-alpha, A-beta, A-delta, B, or C-fibers of the first nerve.

Useful for understanding the invention, disclosed herein are methods for treating migraine and headache conditions. The methods can include, or consist essentially of any number of the following: positioning a first peripheral nerve effector on a patient's skin on an upper extremity of the patient to stimulate a first peripheral nerve selected from the group consisting of one of a median nerve, radial nerve, and ulnar nerve of the patient; positioning a second peripheral nerve effector on or in the ear of the patient to stimulate a second peripheral nerve associated with a parasympathetic nervous pathway of the patient, such as the ABVN; delivering a first electrical nerve stimulation signal transcutaneously to the first peripheral nerve effector to stimulate the first peripheral nerve sufficient to modify at least one neural pathway associated with migraine or headache conditions; and delivering a second electrical nerve stimulation signal transcutaneously to the second peripheral nerve effector to stimulate the second peripheral nerve sufficient to modify at least one neural pathway associated with migraine or headache conditions. The first electrical nerve stimulation signal and the second electrical nerve stimulation signal can be configured to balance parasympathetic and sympathetic nervous system activity of the patient. The method can also include monitoring sympathetic and parasympathetic activity in the patient. The method can also include adjusting the first electrical nerve stimulation signal upon identifying abnormal sympathetic activity in the patient. The method can also include adjusting the second electrical nerve stimulation signal upon identifying abnormal parasympathetic activity in the patient. Third, fourth and additional signals are used in one embodiment.

Also disclosed herein in some embodiments is a system (including but not limited to a wearable system) for treating migraine and optionally other headache conditions. The system can include, or consist essentially of any number of the following features, or others disclosed elsewhere in the specification. The system can include a first peripheral nerve effector configured to be positioned on a patient's skin on an extremity of the patient; a second peripheral nerve effector configured to be positioned on or in the ear of the patient; and/or at least one biomedical sensor or data input source configured to provide feedback information. The controller can be configured to generate a first electrical nerve stimulation signal transcutaneously to the first peripheral nerve effector to stimulate a first peripheral nerve sufficient to modify at least one neural pathway associated with migraine or headache conditions. The controller can also be configured to generate a second electrical nerve stimulation signal transcutaneously to the second peripheral nerve effector to stimulate a second peripheral nerve associated with a parasympathetic nervous pathway of the patient, such as the ABVN, to modify at least one neural pathway associated with migraine or headache conditions. The controller can also be configured to adjust the first electrical nerve stimulation signal and the second electrical nerve stimulation signal to balance parasympathetic and sympathetic nervous system activity of the patient. The controller can be configured to adjust the first electrical nerve stimulation signal upon identifying abnormal sympathetic and/or parasympathetic activity in the patient.

Useful for understanding the invention, disclosed herein is a method for treating migraine and headache conditions. The method can include, or consist essentially of any number of assessing at least one of sympathetic and parasympathetic activity of a subject and determining the presence of abnormal sympathetic or parasympathetic activity in the subject; stimulating a first nerve associated operably connected to the brachial plexus sufficient to have a therapeutic effect on migraine or other headache conditions if abnormal sympathetic activity is present; and stimulating the ABVN sufficient to have a therapeutic effect on migraine or other headache conditions if abnormal parasympathetic activity is present. Stimulation can be in some cases only electrical transcutaneous stimulation, can include exciting or inhibiting nerve activity of the first nerve. Stimulating can involve both the first nerve and the ABVN, if both abnormal sympathetic activity and abnormal parasympathetic activity are present. Assessing at least one of sympathetic and parasympathetic activity of a subject comprises measuring HRV in the subject, such as with a wrist-worn device, and also include measuring heart rate and/or electrodermal activity. The first nerve can be, for example, the median, radial, ulnar, median cutaneous, lateral cutaneous, or other nerves as discussed herein.

Useful for understanding the invention, disclosed herein are methods of treating migraine or other headache conditions, that can include, or consist essentially of electrically stimulating a first peripheral nerve; assessing at least one of sympathetic and parasympathetic activity of a subject and determining abnormal sympathetic or parasympathetic activity in the subject; and adjusting the electrical stimulation based upon assessing the at least one of sympathetic and parasympathetic activity. Adjusting the electrical stimulation can include identifying abnormal sympathetic or parasympathetic activity in the patient, and adjusting the frequency of stimulation of the first nerve, and/or discontinuing electrical stimulation of the first nerve; and initiating electrical stimulation of a second nerve.

Also disclosed herein are systems and methods (the methods being useful for understanding the invention) for providing neurostimulation of one, two, or more peripheral nerve targets that modulate vagal tone, such as the auricular branch of the vagus nerve (ABVN), in which the stimulation is activated in phase with a portion of the respiration cycle by measurements from a sensor that detects events or phases of the respiratory cycle.

Useful for understanding the invention, some examples include, or consist essentially of a method of treating migraine or other headache conditions using combination pharmacotherapy and transcutaneous electrical stimulation, that include any number of the following: administering an amount of one, two, or more pharmacologic agents, including but not limited to triptans, ergots, CGRP-inhibitors, and combinations thereof to a patient; positioning a first peripheral nerve effector on a patient's skin on an extremity of the patient; and delivering a first electrical nerve stimulation signal transcutaneously to the first peripheral nerve effector to stimulate a first peripheral nerve sufficient to modify at least one neural pathway associated with migraine or headache conditions. The triptans can be, for example, one or more of sumatriptan (Imitrex), rizatriptan (Maxalt), almotriptan (Axert), naratriptan (Amerge), zolmitriptan (Zomig), frovatriptan (Frova) and eletriptan (Relpax); the ergots can be, for example, Ergotamine and caffeine combination drugs (Migergot, Cafergot) or Dihydroergotamine (D.H.E. 45, Migranal); the CGRP-inhibitors can be erenumab-aooe (Aimovig), eptinezumab (ALD403), fremanezumab (TEV-48125), or galcanezumab (LY2951742).

Also disclosed herein is a combination pharmacotherapy and electrical stimulation system for treating migraine and optionally other headache conditions, that can include, for example, or consist essentially of any number of features as disclosed in the specification. The system can include, for example, or consist essentially of a device (such as a wearable device) that includes a controller; a first peripheral nerve effector configured to be positioned on a patient's skin on an extremity of the patient; and at least one biomedical sensor or data input source configured to provide feedback information. The controller can be configured to generate a first electrical nerve stimulation signal transcutaneously to the first peripheral nerve effector to stimulate a first peripheral nerve sufficient to modify at least one neural pathway associated with migraine or headache conditions. The system can also include a triptan, ergot, CGRP-inhibitor, or combination thereof for administration to the patient.

In several embodiments, the embodiments described herein that selectively target one or more fiber types of a peripheral nerve and/or that coordinate stimulation of multiple peripheral nerves such that the action potentials reach the same target location (e.g., in the brachial plexus) at the same time or substantially the same time can have one or more of the following advantages: greater therapeutic benefit with less discomfort; less current use (e.g., less power and improved battery life); increased likelihood of patient compliance due to the foregoing. In several embodiments, the embodiments described herein that include multiple peripheral nerve stimulation to promote sympathovagal balance with at least one peripheral nerve modulating the sympathetic nervous system and at least one peripheral nerve modulating the parasympathetic nervous system can advantageously have the ability to selectively modulate either sympathetic and/or parasympathetic arms of the autonomic nervous system in response to detected sympathetic and/or parasympathetic overactivity. In several embodiments, peripheral nerve stimulation can advantageously have synergistic effects when combined with pharmacotherapy, including triptans, ergots, or CGRP-inhibitors. The effects can include enhanced response to therapy, a lesser dose of triptans, ergots, or CGRP-inhibitors needed to achieve the effects and thus lower adverse reactions, and the like. Combination therapy can be beneficial in some embodiments to reduce the time it takes to achieve a therapeutic effect (e.g., by at least 10%, 25%, 50% or more, or overlapping ranges therein) or lengthens the therapeutic effect (by e.g., by at least 10%, 20%, 40% or more, or overlapping ranges therein), or improve the overall benefit (e.g., larger reduction in migraine symptom magnitude or frequency).

In some embodiments, a device is provided that adjusts stimulation modalities to enhance efficacy based on prior responses by the subject and/or predetermined characteristics or features of the neurological or physiologic data measured from the sensors. In some embodiments, one or more sensors are provided to adjust the parameters of the operating mode. This is beneficial in some embodiments to reduce the time it takes to achieve a therapeutic effect (e.g., by at least 10%, 25%, 50% or more, or overlapping ranges therein) or lengthens the therapeutic effect (by e.g., by at least 10%, 20%, 40% or more, or overlapping ranges therein), or improve the overall benefit (e.g., larger reduction in migraine symptom magnitude or frequency).

Alternatively or in addition, and not to be limited by theory, therapy, including stimulation as disclosed herein can invoke a neurohormonal response. Neurohormonal responses can include changes (increase or decrease) in production of norepinephrine, epinephrine, acetylcholine, and/or inflammatory cytokines. Inflammatory cytokines can include interleukin, high-mobility group-box 1 protein, and/or tumor necrosis factor alpha. Neurohormonal response can also be invoked by afferent and/or efferent nerve stimulation of median, radial, ulnar, or vagus nerve, cutaneous nerves or sympathetic nerves. In one embodiment, one or more of norepinephrine, epinephrine, acetylcholine, and/or inflammatory cytokines are reduced post treatment with the devices disclosed herein by at least about 5%, 10-20%, 20-40%, 40-60% or more (including overlapping ranges therein) compared to pre-treatment.

In some embodiments, the systems and methods (the methods being useful for understanding the invention) are configured to treat migraine symptoms, but are not configured to, or do not treat tremors or a movement disorder, including but not limited to Parkinson's disease. In some of the embodiments described herein, one, several or all of the following features are not included: (i) sensors configured to assess patient motion and/or collect motion data, (ii) accelerometers, gyroscopes, magnetometers, inertial measurement units. and (iii) EMG or other muscle sensors. In some embodiments, systems are not configured for, or are not placed on the upper arm and/or are not configured for neuromodulation on the skin surface of the forehead. In some embodiments, systems are not configured to, or do not modulate descending (e.g., efferent) nerve pathways, and only modulate ascending (e.g., afferent) nerve pathways. In some embodiments, systems are not configured to, or do not modulate nerves only on the ventral side of the wrist. In some embodiments, systems do not include any implantable components. In some embodiments, systems are not configured for percutaneous or subcutaneous stimulation, and are only configured for transcutaneous neuromodulation. In some embodiments, systems are not configured for, or do not treat cardiac disorders, including but not limited to hypertension, angina pectoris, ischemia, myocardial infarction, congestive heart failure, and/or arrhythmias. In some embodiments, systems are not configured for only neuromodulating, e.g., stimulating the ventral side of the wrist, rather some configurations may neuromodulate, e.g., deliver stimulation between two or more of the ventral, dorsal, and/or lateral sides of the wrist to target the medial nerve.

In some embodiments, systems are not configured to, or do not stimulate any number of the following acupuncture points: the top of the head (Du 20 Baihui), the forehead (GB.14 Yangbai), behind the ear (GB.20 Fengchi), above the ear (GB.8 Shuaigu), dorsal-side of lower arm (SJ.5 Waiguan), top of the hand between the thumb and index finger (LI.4 Hegu), the toe (GB.41 Linqi), the ankle (GB.37 Guangming), top of the foot (St.44 Neiting), the foot (Liv.3 Taichong), (St.8 Touwei), the temple (GB.4 Hanyan), top of the hand between the thumb and index finger (LI.4 Hegu), the elbow (LI.11 Quchi), and the shin (St.36 Zusanli).

In several embodiments, receiving electroencephalogram (EEG) data relating to the patient, and generating parameters of a first burst electrical stimulation signal and/or a second burst electrical stimulation signal at least in part by analyzing the EEG data relating to the patient is particularly advantageous in allowing for customized stimulation based upon the particular aberrant neuron oscillations that can be contributing to the migraine or other headache pathology. In some embodiments, the EEG data may be recorded with a single channel, 2-channel, 4-channel, 8-channel, 16-channel, 32-channel system, or system with more than 32 channels, with one or more channels positioned over pre-determined regions of interest.

In several embodiments, balancing sympathetic activity by decreasing sympathetic and increasing parasympathetic activity utilizing peripheral neuromodulation is particularly advantageous by restoring balance to the autonomic nervous system, thus reducing the burden of symptoms associated with migraine and other headache conditions.

In several embodiments, transcutaneous nerve neuromodulation at the arm and/or wrist (e.g., median, ulnar, and/or radial nerve stimulation), below the upper arm (e.g., distal to the upper arm/elbow), and on two or more of the ventral, dorsal, and lateral sides of the arm and/or wrist can be particularly advantageous to inhibit sympathoexcitatory related increases in blood pressure and premotor sympathetic neural firing in the rostral ventrolateral medulla (rVLM). The median, ulnar, and/or radial nerves, for example, can provide more convergent input into cardiovascular premotor sympathetic neurons in the rVLM.

Disclosed is a method (the method being useful for understanding the invention) for treating a neurological condition using neuromodulation. The method can include any number of the following: positioning a first nerve effector on a skin surface proximate a median nerve of an arm or wrist of a patient; positioning a second nerve effector on a skin surface proximate a nerve other than the median nerve of the arm or wrist of the patient; receiving data relating to the patient, wherein said data is optionally EEG data; generating parameters of a first neuromodulation signal and a second neuromodulation signal, wherein generating parameters comprises analyzing the data relating to the patient; delivering the first neuromodulation signal to the first nerve effector to modulate the median nerve; and delivering the second neuromodulation signal to the second nerve effector to modulate the nerve other than the median nerve, thereby treating said neurological condition.

Disclosed is a method (the method being useful for understanding the invention) for treating a neurological condition comprising any number of the following: positioning a first nerve effector on a patient's skin on an arm or wrist of the patient distal to the upper arm to modulate a first nerve selected from a median nerve, radial nerve, and ulnar nerve of the patient; positioning a second nerve effector on or within the ear of the patient to modulate the auricular branch of the vagus nerve of the patient; delivering a first burst signal to the first nerve effector to modulate the first nerve sufficient to modify at least one brain or spinal cord autonomic feedback loop relating to the neurological condition; and delivering a second burst signal to the second nerve effector to modulate the second nerve sufficient to modify the at least one brain or spinal cord autonomic feedback loop relating to the neurological condition, wherein the first signal and the second signal are configured to balance parasympathetic and sympathetic nervous system activity of the patient.

A disclosed method (the method being useful for understanding the invention) can include any number of the following features: wherein said first and/or said second nerve effector is a peripheral nerve effector, wherein said first and/or said second nerve effector is an electrode, wherein said first and/or said second nerve effector delivers stimulating electrical signals, wherein the neurological condition is a headache such as migraine, wherein the neurological condition is a throbbing sensation, wherein the neurological condition is a pulsing sensation, wherein a third, fourth and/or fifth nerve effector is provided, wherein the first signal has a frequency of about or less than about 5 Hz, wherein the second signal has a frequency of about or more than about 5 Hz, and wherein the method does not utilize any implantable components, and only involves transcutaneous neuromodulation.

A disclosed method for treating migraines using transcutaneous peripheral nerve stimulation (the method being useful for understanding the invention), can include any number of the following: positioning a first peripheral nerve effector on a skin surface proximate a median nerve of an arm or wrist of a patient; positioning a second peripheral nerve effector on a skin surface proximate a nerve other than the median nerve of the arm or wrist of the patient; transcutaneously delivering the first electrical stimulation signal to the first peripheral nerve effector to stimulate the median nerve; and transcutaneously delivering the second electrical stimulation signal to the second peripheral nerve effector to stimulate a nerve other than the median nerve.

In some embodiments, a wearable device for treating migraines using transcutaneous peripheral nerve stimulation can include any number of the following: a first peripheral nerve effector configured to be placed on a skin surface proximate a median nerve of an arm or wrist of a patient; a second peripheral nerve effector configured to be placed on a skin surface proximate a nerve other than the median nerve of the arm or wrist of the patient; and a controller configured to: transcutaneously delivering the first electrical stimulation signal to the first peripheral nerve effector to stimulate the median nerve; and transcutaneously delivering the second electrical stimulation signal to the second peripheral nerve effector to stimulate a nerve other than the median nerve.

Disclosed is a method for treating or preventing headaches using transcutaneous peripheral nerve stimulation (the method being useful for understanding the invention), comprising any number of: positioning a first peripheral nerve effector on a skin surface proximate a first nerve of an arm or wrist of a patient; and transcutaneously delivering the first electrical stimulation signal to the first peripheral nerve effector to stimulate the first nerve.

In some embodiments, disclosed is a wearable device for treating or preventing headaches using transcutaneous peripheral nerve stimulation, comprising any number of a first peripheral nerve effector configured to be placed on a skin surface proximate a median nerve of an arm or wrist of a patient; and a controller configured to: transcutaneously delivering the first electrical stimulation signal to the first peripheral nerve effector to stimulate the median nerve; and transcutaneously delivering the second electrical stimulation signal to the second peripheral nerve effector to stimulate a nerve other than the median nerve.

Disclosed is a method (the method being useful for understanding the invention) for treating a neurological condition using neuromodulation, comprising any number of: positioning a first nerve effector on a skin surface proximate a median nerve of an arm or wrist of a patient; positioning a second nerve effector on a skin surface proximate a nerve other than the median nerve of the arm or wrist of the patient; receiving data relating to the patient; generating parameters of a first neuromodulation signal and a second neuromodulation signal, wherein generating parameters comprises analyzing the data relating to the patient; delivering the first neuromodulation signal to the first nerve effector to modulate the median nerve; and delivering the second neuromodulation signal to the second nerve effector to modulate the nerve, thereby treating said neurological condition.

### BRIEF DESCRIPTION OF THE DRAWINGS

Non-limiting novel features of the invention are set forth with particularity in the claims that follow. A better understanding of the features and advantages of some embodiments of the present invention will be obtained by reference to the following detailed description that sets forth illustrative embodiments, in which the principles of the invention are utilized, and the accompanying drawings of which:
FIGS. 1A-1D illustrate various views of an embodiment of a device and system that provides peripheral nerve stimulation, targeting individual nerves, to prevent or treat migraine and optionally other headache conditions.
FIG. 1E illustrates a block diagram of an embodiment of a device and system that provides peripheral nerve stimulation and senses a biological measure that is used to customize or modify the delivery of an electrical stimulus.
FIGS. 1F, 1G, and 1H schematically illustrate relationships between select peripheral nerves, spinal cord levels, and neural pathways associated with migraine and other headache conditions.
FIG. 2A illustrates an embodiment of a stimulator with electrodes that can be disposed on a wearable band, according to some embodiments of the invention.
FIGS. 3A and 3B illustrate various embodiments of a monitoring unit and a therapy unit that form a two-part treatment system.
FIG. 3C schematically illustrates selected anatomy relating to the ear, including the area innervated by the auricular branch of the vagus nerve (ABVN).
FIG. 3D illustrates an embodiment of an auricular stimulation device.
FIGS. 4A-4D illustrate an embodiment of a two-part system with a single monitoring unit and a plurality of therapy units.
FIG. 4E schematically illustrates an embodiment of an EEG system from which data can be recorded from one or more predetermined regions of interest.
FIGS. 5A-5I illustrate embodiments of a wearable therapy system.
FIG. 6 illustrates an embodiment of the wearable therapy system that uses the cloud to receive and transmit data between the therapy system, a secondary patient device, and a physician.
FIG. 7 is a block diagram that illustrates the individual components of the therapy unit, band, and base station shown in FIG. 6.
FIGS. 8A-8B illustrate human body meridian points that can be used as locations for stimulation.

### DETAILED DESCRIPTION

Not to be limited by theory, migraine is a brain disorder of dysregulation of sensory processing associated with autonomic involvement, and triggers can be driven by an imbalance of autonomic activity; that is an imbalance of sympathetic and parasympathetic activity within the autonomic nervous system. This imbalance can arise from overactivity or underactivity of the sympathetic and/or parasympathetic limbs of the autonomic nervous system. Electrical stimulation that affects the autonomic nervous system including systems and methods as disclosed herein (the methods being useful for understanding the invention) can provide therapeutic benefit by restoring balance to the autonomic nervous system, thus reducing the burden of symptoms associated with migraine and other headache conditions. Not to be limited by theory, migraine can be a disorder of chronic sympathetic dysfunction in an anatomically intact system. Sympathetic dysfunction can be related to an imbalance of co-transmitters. Prolonged stimulation of the sympathetic nervous system depletes norepinephrine and increases release of other co-transmitters. Migraine symptoms can be caused by overactivation of the sympathetic nervous system, leading to differential release of co-transmitters. Thalamocortical dysrhythmia can be an underlying cause of migraine, which can be observed with EEG measurements as low frequency cortical oscillations. During interictal periods, resting state EEG of migraineurs tends to shift from alpha range (e.g., 8-12 Hz) to theta range (e.g., 4-8 Hz). These aberrant neuronal oscillatory patterns can be responsible for the symptoms that accompany migraine both ictally and/or interictally such as photo/phonophobia and vertigo. Deprivation of sensory input leads to a thalamocortical column-specific decrease in information processing, which permits the slowing of resting-state thalamocortical activity from alpha to theta frequencies, as less information needs to be processed. Median nerve neuromodulation (e.g., stimulation), for example, can provide direct, synaptically-mediated sensory input to the thalamocortical circuit to increase information processing. Patterned or bursting stimulation of the median or other nerves can advantageously modulate the resting state cortical oscillation frequency to a more normal, healthy state, such as from theta range to the alpha range. Sympathetic overactivation may be an underlying cause of migraine (see attached slide). Patterned or bursting stimulation of the median nerve can reduce sympathetic outflow at both acute timepoints (e.g., about 30 minutes) and chronic timepoints (e.g., about 2 weeks). Therapeutic benefit improves over time with repeated daily usage, so treatment can be used for both symptomatic relief and as a prophylactic. Multimodal allodynia and hyperalgesia beyond the locus of migraine headache is mediated by sensitized thalamic neurons that process nociceptive information from the cranial meninges together with sensory information from the skin of the scalp, face, body, and limbs. Patterned stimulation of the one or more peripheral nerves that project to the thalamus (e.g., median, radial, ulnar, etc.) can attenuate sensitization by providing additional sensory input to disrupt, block, or mediate nociceptive information from the cranial meninges that trigger migraine.

Also not to be limited by theory, vagal nerve stimulation can modulate the trigeminal nuclei to inhibit inflammation. Hypersensitization of the brainstem trigeminal sensory complex may underlie the primary brain dysfunction in migraine by upregulation of cortical excitability. Vagal afferents project to nucleus tractus solitarii (NTS) and spinal trigeminal nucleus (Sp5), regions that modulate trigeminal sensory complex excitability and connectivity with higher brain structures. Trigeminal sensory nuclei can be involved in neurogenic inflammation during migraine characterized by vasodilation. VNS can modulate the trigeminal sensory pathway to ameliorate migraine pathophysiology and reduce headache frequency and severity. Increased activation of raphe nuclei and locus coeruleus may inhibit nociceptive processing in the sensory trigeminal nucleus. Human skin is well innervated with autonomic nerves and neuromodulation (e.g., stimulation) of nerve or meridian points as disclosed herein can potentially help in treatment of migraine or other headache conditions. For example, afferent nerves in the periphery or distal limbs, including but not limited to median nerve, are connected by neural circuits to the arcuate nucleus of the hypothalamus.

Alternatively or in addition, median nerve stimulation can regulate the orexinergic pathway for coma, and which is a neural pathway associated with the onset and treatment of migraine. Activation of the orexinergic network can also be used to treat migraine headache. Alternatively or in addition, and not to be limited by theory, antidromic stimulation of autonomic or visceral efferent nerve fibers in the arm, leg, neck, or tragus may modulate sympathetic outflow and/or modulate vagal tone. Specifically, sympathetic afferents and/or efferents can be specifically modulated, e.g., stimulated by targeting c-fibers in the periphery of the body.

Alternatively or in addition, and not to be limited by theory, electrical neuromodulation, e.g., stimulation of peripheral nerves, either somatic, autonomic, afferent, and/or efferent, can reduce susceptibility to migraine attacks.

Alternatively or in addition, respiratory gated auricular stimulation has been demonstrated to target brain networks involved in migraine and shows therapeutic promise. In some embodiments, systems for providing neurostimulation of one, two, or more peripheral nerve targets that modulate vagal tone, parasympathetic outflow, vagal brainstem regions, sympathetic outflow, or sympathetically mediated brainstem regions, in which the stimulation is activated in phase with a portion of respiration by measurements of the respiratory cycle. In particular, the systems can use a detecting device to detect respiration cycles over time. When a predetermined relationship or correlation between the detected activity and a threshold value, such as a match, rate of change in activity, or within a predefined range, a stimulator is instructed to provide stimulation to at least one or more peripheral nerves. The stimulation can be advantageously correlated to the detected respiration phase, such as exhalation, providing potential synergistically increased effect of the stimulation and thus improved therapeutic benefit.

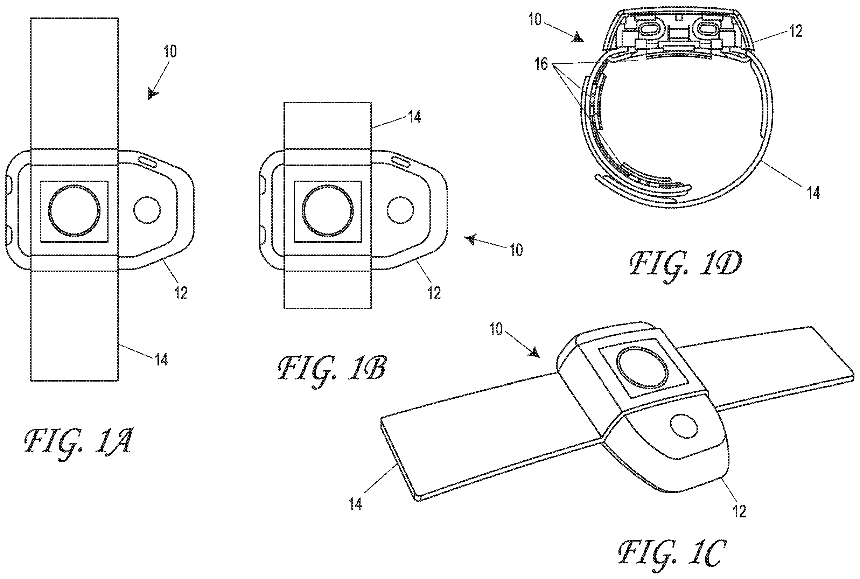

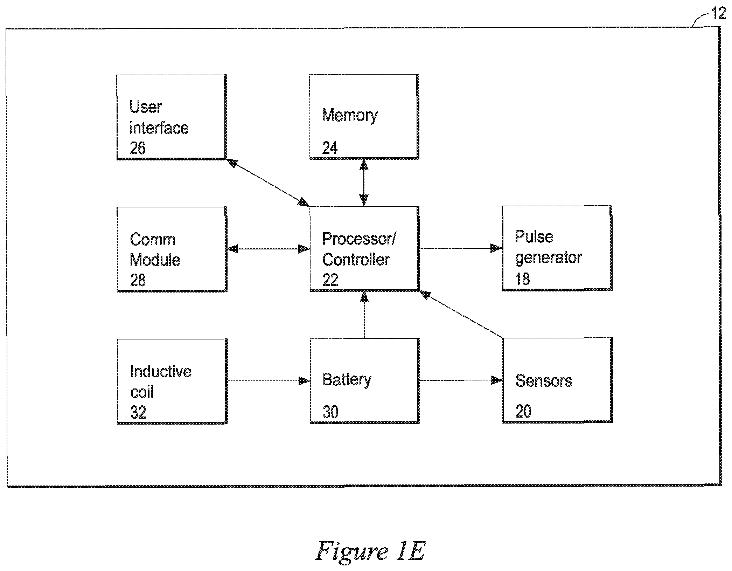

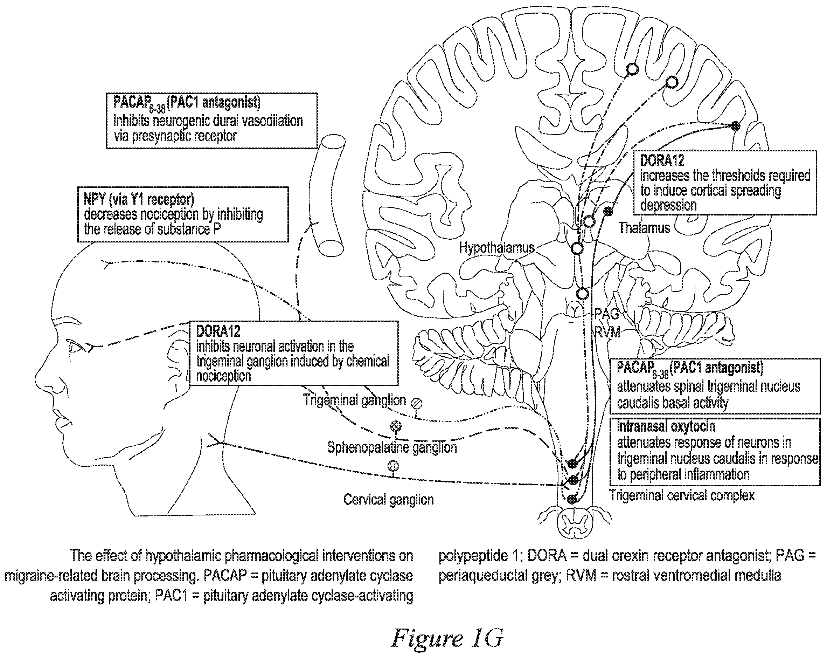

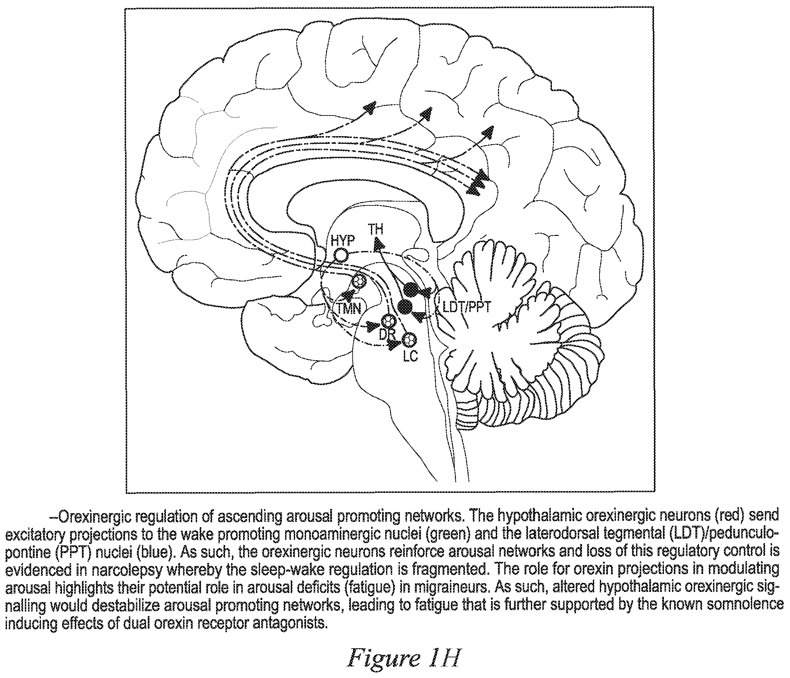

Some embodiments, as shown in FIGS. 1A-1E for example, are related to a device and system that provides peripheral nerve stimulation, targeting individual nerves. Some embodiments involve a device and system 10 that allows customization and optimization of electrical treatment to an individual. In particular, the device 10 described is configured for electrical stimulation of the median, and optionally radial, ulnar, peroneal, saphenous, tibial and/or other nerves or meridians accessible on the limbs for treating migraine and other headache conditions. Targeting those specific nerves and utilizing appropriately customized stimulation results in more effective therapy. In some embodiments, useful for understanding the invention, therapy can reduce or eliminate the number, dose, and/or frequency of medications that a patient may need to take for their chronic migraine or headache condition, advantageously reducing side effects/potential toxicities. In some embodiments, useful for understanding the invention, therapy can have an unexpectedly synergistic effect when combined with one, two, or more pharmacologic agents such as a triptan, ergot, or CGRP-inhibitor. In some embodiments, a triptan, ergot, or CGRP-inhibitor can be administered orally, intravenously, or another route along with peripheral nerve stimulation protocols such as described herein for an unexpected synergistically beneficial effect in treating migraine or other headache conditions.

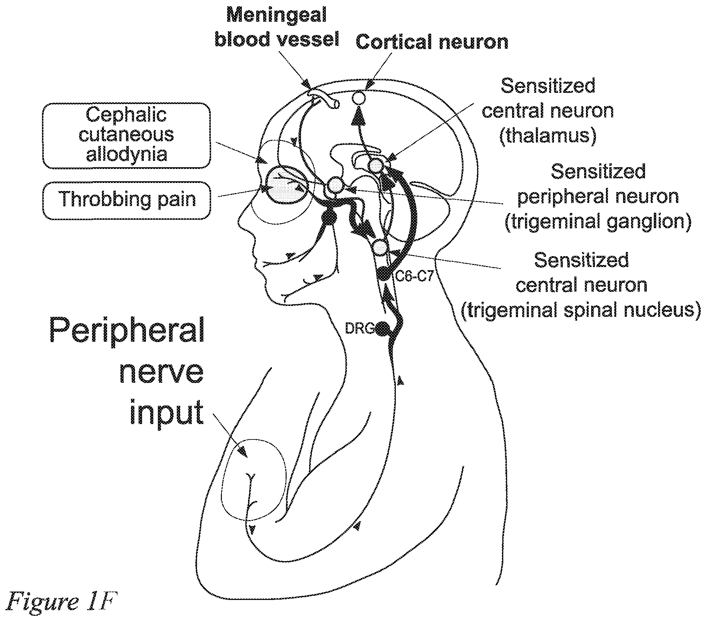

Afferent nerves in the periphery or distal limbs, including but not limited to the median nerve, are connected via neural pathways to sensitized peripheral and central neuron connected to the hypothalamus, as illustrated schematically in FIGS. 1F-1H.

FIGS. 1A-1E illustrate an embodiment of a device and system 10 that provides transcutaneous peripheral nerve neuromodulation, e.g., stimulation, targeting one, two, three, or more individual nerves, to treat migraines and optionally other headache conditions. In some embodiments, the device 10 is designed and configured to be worn on the wrist or arm. In some embodiments, the device 10 is configured for placement below the upper arm/elbow. In some embodiments, electronics located in a watch-like housing 12 measure heart rate, motion, and/or electrodermal activity, and also generate an electrical stimulation waveform. Electrical contacts in a band 14 and/or housing 12 transmit the stimulation waveform to the disposable electrodes 16. The location of the contacts in the band 12 is arranged such that one or more specific nerves are targeted at the wrist, such as the median, radial, and/or ulnar nerves. The electronics housing 12 also can have a digital display screen to provide feedback about the stimulation and sensor measurements, derived characteristics and history to the wearer of the device.

In some embodiments, the treatment device 10 is a wrist-worn device that can include, for example, 1) an array of electrodes 16 encircling the wrist, 2) a skin interface to ensure good electrical contact to the person, 3) an electronics box or housing 12 containing the stimulator or pulse generator 18, sensors 20, and other associated electronics such as a controller or processor 22 for executing instructions, memory 24 for storing instructions, a user interface 26 which can include a display and buttons, a communications module 28, a battery 30 that can be rechargeable, and optionally an inductive coil 32 for charging the battery 30, and the like, and/or 4) a band to hold all the components together and securely fasten the device around the wrist of an individual.

In FIG. 1D, electrodes 16 are placed circumferentially around the wrist and neuromodulated, e.g., excited on opposite sides of the wrist, the electric field extends through the wrist and this enables excitation of nerves deeper in the tissue. Therefore, the circumferential array is compact, allowing a band width that is approximately the same size as the electrode width, and thus advantageous for wearable devices. In some embodiments, the advantage of having the configurability of the array is that the same nerves can be reached, but in a more compact form factor than conventional median nerve excitation. The devices described herein may be described and illustrated with electrodes placed circumferentially or longitudinally, but it should be understood that either electrode configuration can be used by the devices. In addition, the devices may be described and shown with 2, 3 or more electrodes, but it should be understood that the device can have only 2 electrodes, or can have more than 2 electrodes. Some devices may be designed to stimulate just a single nerve, such as the median nerve, and some devices may be designed to stimulate more than one nerve.

Not to be limited by theory, transcutaneous nerve neuromodulation at the arm and/or wrist (e.g., median and/or radial nerve stimulation) can advantageously inhibit sympathoexcitatory related increases in blood pressure and premotor sympathetic neural firing in the rostral ventrolateral medulla (rVLM). The median and/or radial nerves, for example, can provide more convergent input into cardiovascular premotor sympathetic neurons in the rVLM.

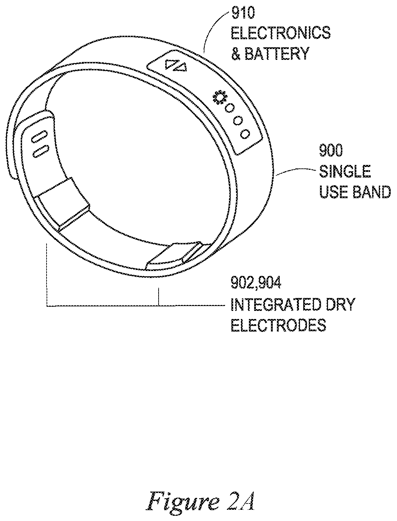

FIG. 2A shows an embodiment of a wearable band 900 with integrated electrodes 902, 904. The integrated electrodes 902, 904 can be dry electrodes in electrical communication with a detachable controller 910 through a flexible circuit embedded in the band. In some cases, dry electrodes may be more suitable for longer term use electrodes that can be used for months, such as at least 1, 2, or 3 months, before the band needs to be replaced. In some embodiments, the band may be a single use band that can be used for a relatively long period of time before replacement.

Embodiments of the invention can include a device and system to measure and collect biological data (e.g., heart rate, heart rate variability, ECG, galvanic skin response, temperature, and/or blood pressure), analyze the data as to interpret how these measures may influence migraine or other headache conditions, and provide peripheral nerve stimulation that targets one or more individual nerves, such as the median, ulnar, radial, and/or ABVN, to treat or prevent migraine or other headache conditions, where the stimulation applied may or may not be modified based on the measured data.

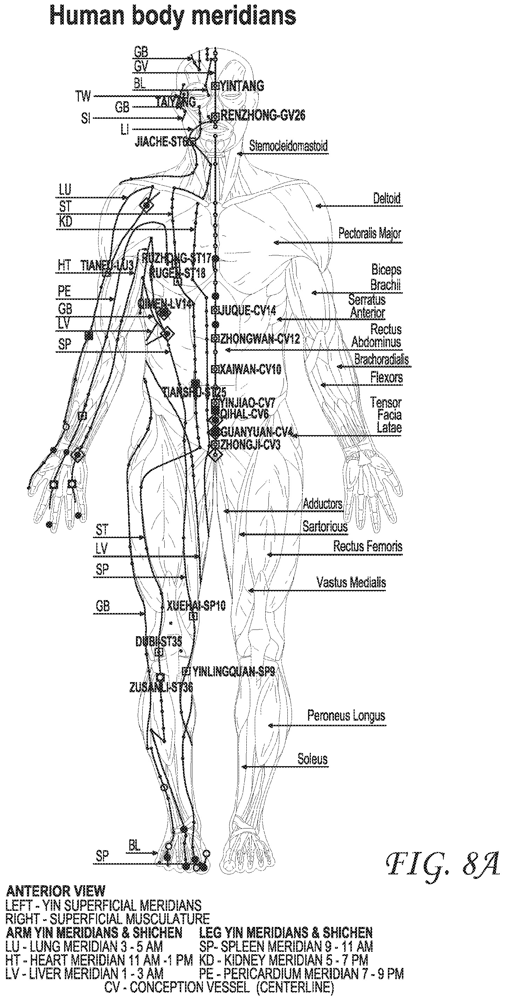

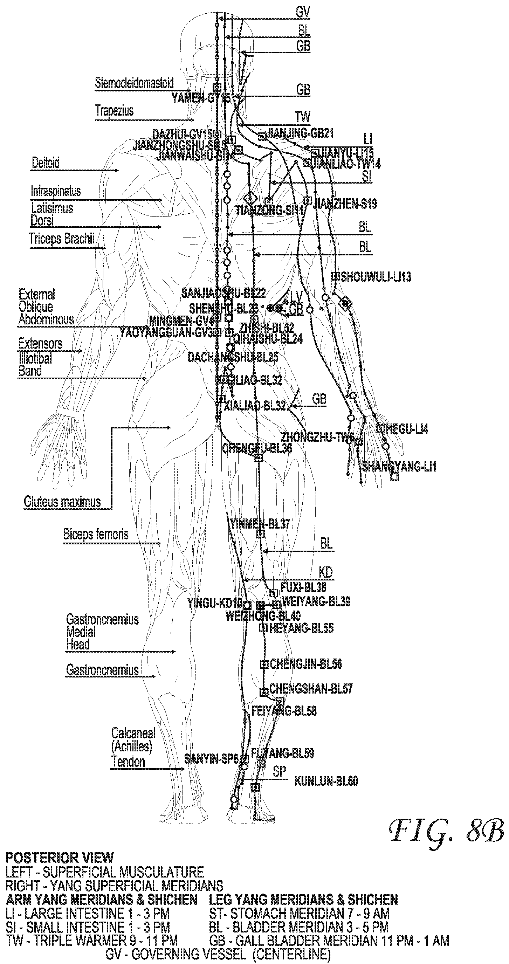

Embodiments of the therapy system can be flexible or adaptable to be, instead of, or in addition to the arm or the wrist, worn on various locations of the body to access a specific nerve, such as the median nerve at the wrist or elbow or the saphenous, or the ABVN in the ear, or tibial nerves near the knee or ankle for example; or various nerves, such as the radial and/or ulnar nerves, or various acu-pressure or meridian points as shown in FIGS. 8A and 8B.

In some embodiments, the electrodes can be positioned for myofascial innervation, preferably near the Neiguan or PC 5-6 or PE5 or PE6 acupressure point, which is about 3 finger widths proximally from the wrist crease. Alternatively, the electrodes can be positioned distally on the arm, where the median nerves are closer to the skin surface, which requires less power and can provide a more comfortable transcutaneous simulation.

Embodiments of the therapy system can include any number of the following three components: (1) a monitoring unit having sensors, circuitry, and optionally may have a power source and/or a microcontroller, (2) a therapy unit having a stimulator (e.g., a pulse generator), circuitry, a power source and a microcontroller, and (3) a skin interface having electrodes and electrical connections for electrically connecting the electrodes to the therapy unit. In some embodiments, all three components are separate components that can be reversibly attached to each other to form a wearable therapy system. In some embodiments, any two of the components can be combined or integrated together to form a wearable two-part system that can be reversibly attached to each other. It should be noted that some functions can crossover, such as the electrodes of the skin interface being used as sensors to measure electrical activity (e.g. EMG and ECG) and impedance, for example. In some embodiments, any one of the detachable components can be disposable and/or can be sent back to the manufacturer for recycling. In some embodiments, the sensor can be separate, such as a chest mounted effort belt to measure respiration phase and rate, which can wirelessly communicate with the stimulator.

In some embodiments, some or all of components of the therapy system can be implantable, percutaneous, and/or transcutaneous. For example, the stimulation electrodes may be implanted in the vicinity of a target nerve. Electrical power can be delivered to the electrodes via a wired connection or wirelessly. Implanted electrodes can have various shapes to direct the flow of current toward the target nerve, including but not limited to a nerve cuff or electrodes that might be cylindrical or flat (plate shaped). The stimulation electrodes can also be inserted percutaneously or transcutaneously. Alternatively, sensors can be implanted in a location such that they are able to continuously measure electrical activity, like in the chest or in the wrist, percutaneous, and/or transcutaneous. Implanted components can also communicate with other components of the therapy system via wired connection or wirelessly.

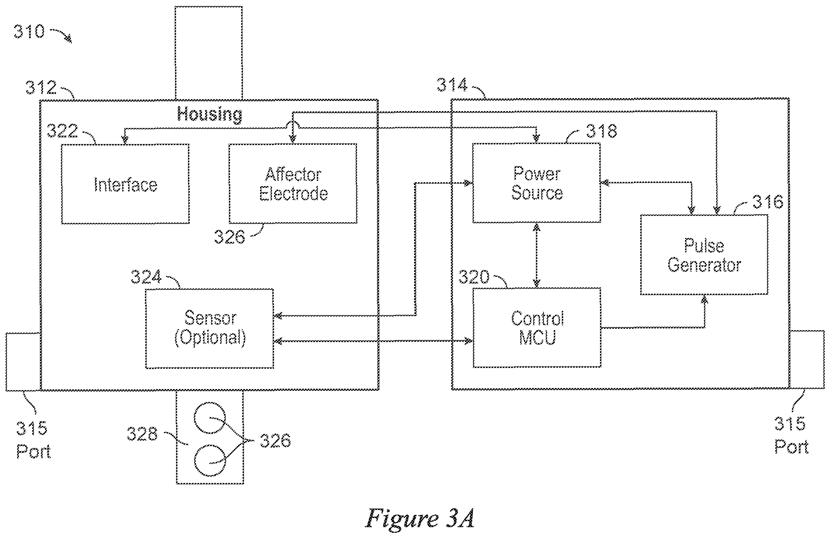

One embodiment, as shown in FIG. 3A, is a two-part system 310 including a monitor unit 312 that can be wearable in some embodiments and a therapy unit 314. In some embodiments, the therapy unit 314 can be can be detachable and can be reversibly attached to the wearable monitor unit 312. The therapy unit 314 may contain an electrical stimulation signal generator 316, power source 318, and a microprocessor and/or microcontroller 320 to control the stimulation. The therapy unit 314 can reversibly connect and communicate directly and/or wirelessly to the wearable monitor 312. In some embodiments, the therapy unit 314 may remain separate from the wearable monitor unit 312 and can communicate wirelessly with the wearable monitor unit 312. In some embodiments, the therapy unit 314 can have a data/power port 315, such as a USB port that allows a user to charge the power source 318, update the software and/or parameters on the microcontroller 320, and/or retrieve data from memory on the wearable monitor unit 312 and/or therapy unit 314. In some embodiments, the data/power port can be located on the wearable monitor unit 312 or both the wearable monitor unit 12 and therapy unit 314. In some embodiments, the wearable monitor unit 312 and/or therapy unit 314 can communicate wirelessly with an external computing device to update the software and/or parameters and/or retrieve data.

In some embodiments, the wearable monitor unit 312 can have a housing with a user interface 322 that encloses one or more sensors 324. In some embodiments, the wearable monitor 312 can be used to measure heart rate, rhythm, heart rate variability (HRV), or other measures correlated or related to migraine, other headache conditions, or response of the autonomic nervous system. In some embodiments, the wearable monitor 312 can have one or more electrodes 326 located on the base of the housing that makes contact with the patient's skin. In addition or alternatively, the wearable monitor 312 can have a band 328 or other securement feature with one or more electrodes on the skin facing side of the band 328. In some embodiments, the wearable monitor unit 312 has 2 or 3 electrodes, or at least 2 or 3 electrodes. In some embodiments, the wearable monitor unit 312 lacks a power source and relies on the power source 318 in the therapy unit 314 for power. In other embodiments, both the wearable monitor unit 312 and the therapy unit 314 have power sources. In some embodiments, only the wearable monitor unit 312 has a power source and the therapy unit relies on power from the monitoring unit.

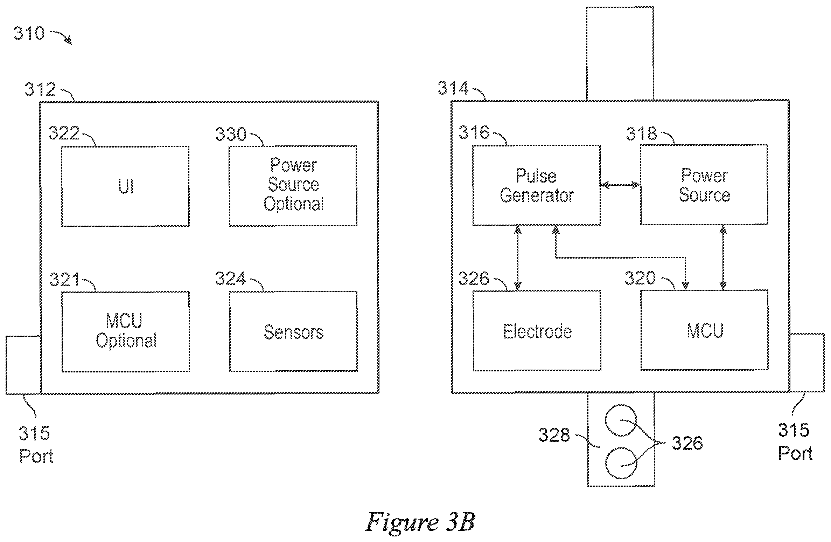

In some embodiments, as shown in FIG. 3B, the therapy unit 314 may directly make contact with the wearer's skin and have the capability to provide electrical stimulation of targeted nerves, such as the median, radial, ulnar, and/or ABVN, using electrodes 326. In some embodiments, the therapy unit 314 has 2 or 3 electrodes, or at least 2 or 3 electrodes. These electrodes 326 may be located on the housing of the therapy unit 314 and/or the therapy unit 314 may also have a band 328 or securement feature with electrodes 326. In some embodiments, when the therapy unit 314 has electrodes 326, the wearable monitor unit 312 does not have electrodes. In some embodiments, both the monitor unit and the therapy unit can have electrodes. As above, the therapy unit 314 can have a stimulator 316, power source 318, and microcontroller 320. The wearable monitor unit 312 can have a user interface 322 and one or more sensors 324 and, optionally, a power source 330 and microcontroller 321. In some embodiments, when the monitor unit has a power source 330 and/or a microcontroller 321, the therapy unit does not have a power source and/or a microcontroller. In some embodiments, the wearable monitor unit 312 is a smart watch or other wearable device, such as the Apple Watch or an Android based smart watch, with an application that allows the wearable device to communicate with the therapy unit and perform as a monitor unit. In some embodiments, the wearable monitor unit 312 can communicate with the therapy unit 314 wirelessly, and one or both of these devices can also communicate with an external computing device wirelessly. In some embodiments, one or both of the wearable monitor unit 312 and the therapy unit 314 can have a data/power port 315. In some embodiments, the wearable monitor unit 312 and the therapy unit 314 can be connected to each other through the data/power ports 315.

In some embodiments, the sensors can be located in or on the therapy unit instead of the monitoring unit. In some embodiments, the sensors can be located on both the therapy unit and the monitoring unit. In some embodiments, one or more sensors can be located on a separate wearable device, such as a sensor on a band that can be worn around the arm, leg, neck, or chest, or a sensor implanted inside the body, which may communicate via a wired or wireless connection with the therapy unit and/or the monitoring unit.

In some embodiments, the monitor unit can instead be carried by the user in, for example, the user's hand or pocket, rather than be worn. For example, a monitor unit carried by the user can be a smart phone, such as an Android smartphone or iPhone.

In some embodiments, the two-part system or the monitor unit may instruct the user to perform an action, such as to sit and relax the arm, or to remain still or to attempt to remain still while the wearable monitor unit takes a measurement with one of the sensors.

In some embodiments, the user interface can include a display. In some embodiments, the display can be a touch screen display or capacitive sensor. In some embodiments, the display can be an array of LED lights. In some embodiments, the user interface can include one or more buttons, a dial, and/or a keyboard.

In some embodiments, the electrodes can be dry-contact (e.g., fabric, metal, silicone or any other plastic impregnated with conductive fillers, or a combination), use a conductive gel (e.g., hydrogels), or have a wet electrode surface (e.g., a sponge with water or conductive liquids or gels), or have fine micro needles, for example. In some embodiments, the electrodes can have a foam backing.

In some embodiments, the monitor unit can be a wearable monitor having a housing with a user interface. The housing can use a plurality of sensors to collect, store, and analyze biological measures about the wearer including, but not limited to, blood pressure, motion (e.g., accelerometers, gyroscopes, magnetometer, bend sensors), muscle activity (e.g., EMG using electrodes), cardiovascular rhythm measures (e.g., heart rate, heart rate variability, or ventricular and/or atrial dyssynchrony using electrodes to measure ECG, heart rhythm abnormalities), skin conductance (e.g., skin conductance response, galvanic skin response, using electrodes), respiratory rate and phase, skin temperature, pupil diameter, and sleep state (e.g., awake, light sleep, deep sleep, REM). Heart rhythm measures can be recorded with optical, electrical, and/or accelerometery-based sensors. In particular, studies have shown that increased stress levels can increase blood pressure. Respiratory measures can be recorded with mechanical, electrical, impedance, acoustic, ultrasonic, infrared, or video based measures. Activities such as exercise, can also affect onset of migraine or headache conditions - measuring accelerometry (motion), heart rate, etc. could help identify these activities and normalize the measurements by similar activities. Thus, using standard statistical analysis, machine learning, deep learning, or big data techniques, such as a logistical regression or Naive Bayes classifier, these biological measures can be analyzed to assess a person's state, such as level of stress, which in turn, can serve as a predictor for migraine or headache attacks. In some embodiments, the device can provide stimulation based on measurements of one or more biological measures, a determination of a person's state, and/or a prediction of migraine or headache attacks.

In some embodiments, the responsiveness of stimulation could be dependent on one, two, or more sensors housed in the device to collect, store, and analyze biological measures about the wearer including, but not limited to, motion (e.g., accelerometers, gyroscopes, magnetometer, bend sensors), ground reaction force or foot pressure (e.g., force sensors or pressure insoles), muscle activity (e.g., EMG), cardiovascular measures (e.g., heart rate, heart rate variability (HRV), photoplethysmography (PPG), or ventricular and/or atrial dyssynchrony using electrodes to measure ECG and/or heart rhythm abnormalities), skin conductance (e.g., skin conductance response, galvanic skin response), respiratory rate, skin temperature, pupil diameter, and sleep state (e.g., awake, light sleep, deep sleep, REM). Using standard statistical analysis, machine learning, deep learning, or big data techniques, such as a logistical regression or a Naive Bayesian classifier, these biological measures can be analyzed to assess the wearer's activity state, such as sedentary versus active, level of stress and the like, which in turn, can serve as a predictor migraine or headache attacks.

Sympathetic and parasympathetic activity can be measured through several methods, including microneurography (MSNA), catecholamine tests, heart rate, HRV, or galvanic skin response. HRV can provide a quick and effective approximation of autonomic activity in the body. HRV can be determined by analyzing the time intervals between heartbeats, also known as RR intervals. Heart rate can be accurately captured, for example, through recording devices such as chest straps or finger sensors. The differences between successive RR intervals can provide a picture of one's heart health and autonomic activity. Generally speaking, healthier hearts have more variability between successive RR-intervals. This interbeat data can also be used to denote an individual's sympathetic and parasympathetic activity levels. Through frequency-domain analysis, heartbeat frequencies can be separated into distinct bands. High-frequency signals (~0.15-0.4 Hz) can almost exclusively reflect parasympathetic activity, and low-frequency signals (~0.04-0.15 Hz) can represent a mixture of sympathetic and parasympathetic activity. Therefore, taking the ratio of high frequency (HF) to low frequency (LF) signals can yield an approximation of one's sympathetic tone. In some embodiments, HRV can be analyzed, for example, under time-domain, geometric domain methods in addition to frequency domain methods. In some embodiments, increased heart rate variability can signify increased parasympathetic response and/or decreased sympathetic response. Decreased heart rate variability can signify decreased parasympathetic response and/or increased sympathetic response. In some embodiments, a system can sense an increase or decrease in HRV of about or more than about 5%, 10%, 15%, 20%, 25%, 30%, 35%, 40%, 45%, 50%, 75%, 100%, or more over a baseline value (or target desired HRV value) and institute a change in one, two, or more stimulation modality parameters accordingly. In some embodiments, the one, two, or more stimulation modalities can be configured to modulate, such as increase or decrease stimulation to one or more nerves (e.g., peripheral nerves) associated with the sympathetic and/or parasympathetic nervous system, and a response to therapy can be confirmed by sensing an increase or decrease in parasympathetic or sympathetic tone, including but not limited to increase or decrease in HRV, changes in high frequency content of HRV, and changes in the ratio of high frequency and low frequency content of HRV. In some embodiments, balance of parasympathetic and sympathetic activity can be assessed with frequency analysis of heart rate variability measured with pulsed plethysmography with an LED light source and optical sensor disposed in the device that measures fluctuations in light level due to blood flow that target one of the major blood vessels around the knee or in the arm or neck or ear in other embodiments. In some embodiments, heart rate could be measured using accelerometer-based sensors or with electrical-based sensors, similar to single or multiple-lead ECG monitors.

A large source of error in optical measurements of heart rate in some cases is motion artifacts due to relative motion between the optical sensor and the blood vessel being measures. In some embodiments, the optical heart rate sensor has an adhesive on the side of housing that contacts the wearer's skin to reduce relative motion between the sensor and the target blood vessel.

HRV measurements in subjects with chronic migraines or headache can be significantly different compared to controls. Through frequency-domain analysis, heartbeat frequencies can be separated into distinct bands. High-frequency signals (between about 0.15 Hz and about 0.4 Hz) can almost exclusively reflect parasympathetic activity, and low-frequency signals (between about 0.04 Hz and about 0.15 Hz) can represent a mixture of sympathetic and parasympathetic activity. In some embodiments, taking the ratio of high frequency (HF) to low frequency (LF) signals yields an approximation of one's sympathetic tone. Very low frequency (VLF) signals (between about 0.004 Hz and about 0.040 Hz) can also be evaluated to assess parasympathetic activity. The total power of HRV in the frequency domain can also be evaluated to assess autonomic activity.

Sympathetic and parasympathetic functions can also be evaluated, for example, by analyzing mean normal-to-normal intervals, e.g., all intervals between adjacent QRS complexes of measured cardiac rhythm, including the number of interval differences of successive NN intervals greater than 50 milliseconds; square root of the mean squared differences of successive NN intervals, and standard deviation of the NN intervals.

In some embodiments, sympathetic activity can also be assessed using more traditional techniques, such as measuring blood pressure changes before release and before starting a hand grip exercise, or measuring blood pressure changes before and after immersing the hand in a bath of cold water (e.g., cold pressor test). Parasympathetic activity can be assessed by measuring heart rate response during deep breathing, or heart rate response to standing from lying or seated position (orthostatics), or by changing the orientation of a person's body using, for example, a tilt table. Both sympathetic and parasympathetic activity can be assessed during the Valsalva maneuver (e.g., blowing into a mercury manometer and maintaining a pressure of about or at least about 40 mmHg), or orthostatic heart rate response (e.g., to standing from lying or seated position).

In some embodiments, one, two, or more additional sensors are disposed in the device, including electrical and/or accelerometer sensors in contact with the wearer's skin to measure cardiac activity or pressure sensors to measure changes in blood vessels, to be used in combination with an optical sensor to improve the fidelity of heart rate measurement.

In some embodiments, the system and device have memory and a processor to extract RR intervals from sensor data, calculate variability of RR intervals, transform data into frequency domain, and calculate high frequency signals, low frequency signals, and the ratio of the high frequency and low frequency signals.

In some embodiments, the heart rate sensor can store collected data for specified time period to gather adequate date for heart rate variability calculation. Specified time period can range in some cases from 1 - 60 seconds, and may extend to 10 minutes or more.

In some embodiments, electrodermal activity, also known as galvanic skin response or skin conductance response, for example, can be measured using sensors, such as electrodes; hereafter, galvanic skin response and electrodermal activity are used synonymously. Galvanic skin response is the change of the electrical resistance of the skin caused by emotional stress, and measurable with a sensitive galvanometer. Not to be limited by theory, skin resistance varies with the state of sweat glands in the skin. Sweating is controlled by the sympathetic nervous system, and skin conductance can be an indication of psychological or physiological arousal. If the sympathetic nervous system is highly aroused, then sweat gland activity also increases, which in turn increases skin conductance. In this way, skin conductance can be a measure of emotional and sympathetic responses, and the feedback data can be sent to the controller, which will in turn modulate stimulation to, for example, decrease sympathetic nervous system activity. Other non-limiting parameters associated with sympathetic and/or parasympathetic nervous system activity that can be sensed include, for example, sweating during particular times of the day and/or night, sleep states as detected, for example, by an EEG sensor, e.g., an EEG headband for example (to determine when sympathetic and/or parasympathetic activity is particularly high or low, and potentially correlating a sleep state such as stage 1, 2, 3, 4, or REM), and/or motion. In some embodiments, a diagnostic and/or combination diagnostic/stimulation device can be configured to measure a person's heart rate and galvanic skin response for improved estimation of the person's autonomic activity; this estimation of autonomic activity can in turn be used to adjust the stimulation applied as treatment, including but not limited to frequency of stimulation, coordination of bursting of stimulation, selected nerve target, duration of stimulation session, or the time of day stimulation is applied. In some embodiments, a wearable device, such as a wrist-worn device can include both electrodermal activity (EDA) sensors and heart rate sensors. This combination of data can in some embodiments advantageously and synergistically provide improved estimation of sympathetic and parasympathetic activity than a single measure alone. In some embodiments, the system can include multiple sensors to measure electrodemal activity in conjunction with heart rate and HRV. Data from the multiple sensors can be analyzed by a hardware or software processor and combined to provide a more accurate estimation of sympathetic and/or parasympathetic activity. In some embodiments, the EDA and HR sensors can be disposed in a wrist-worn device that communicates via a wired or wireless connection to the stimulator or to send data to a centralized remote server (e.g., the cloud). Stimulation parameters, such as frequency or pulse width among others, nerve target locations (e.g., radial, ulnar or ABV nerves for example) or dosing regimen (e.g., duration or time of day of stimulation sessions) could be adjusted based on estimations of sympathetic and/or parasympathetic activity. In some embodiments, significant changes in sympathetic and/or parasympathetic activity can be used to predict the onset of migraine or headache, and the device can start stimulation to prevent or reduce the duration of the episode. Adjustments could be made in real-time, or in subsequent stimulation sessions. In some embodiments, stimulation frequency can be adjusted to either increase or decrease autonomic activity modulated by a single specific nerve, or multiple nerves. For example, in some embodiments, relatively low frequency stimulation of a target nerve (e.g., below a threshold value, e.g., about 5 Hz) can potentially inhibit the nerve and thus decreases sympathetic activity, while higher frequency stimulation (e.g., above a threshold value, e.g., about 5 Hz) can potentially excite the nerve and thus increases sympathetic activity. In some embodiments, the stimulation frequency can be, for example, less than about 20 Hz, 15 Hz, 10 Hz, 5 Hz, 4 Hz, 3 Hz, 2 Hz, 1 Hz, or less, or ranges including any two of the foregoing values. Additionally, pulse width of the stimulation waveform can be adjusted to recruit more or less of a specific fiber type, including cutaneous fibers, which can inhibit sympathetic activity. The same effect can occur with the same or other target nerves to regulate parasympathetic activity. In other words, in some embodiments, relatively low frequency stimulation of the target nerve (e.g., below a threshold value, e.g., about 5 Hz) can potentially inhibit the nerve and thus decreases parasympathetic activity, while higher frequency stimulation (e.g., above a threshold value, e.g., about 5 Hz) can potentially excite the nerve and thus increases parasympathetic activity. Not to be limited by theory, depending on the stimulation parameters for example, in some cases stimulating the target nerve can increase or decrease either sympathetic activity, parasympathetic activity, or both. In some embodiments, stimulation of the saphenous nerve can affect sympathetic activity, and stimulation of the tibial nerve can affect parasympathetic activity.

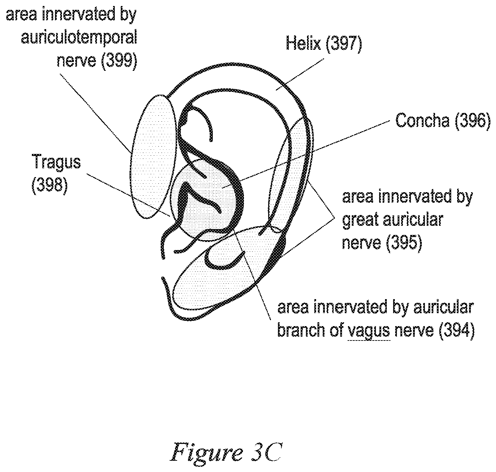

In some embodiments, stimulation of one, two, or more nerves in the upper and/or lower extremity can be combined with stimulation of the ABVN, such as by way of the cymba concha or tragus, to modulate vagal activity and restore balance of the autonomic nervous system. In an alternative embodiment, useful for understanding the invention, the system can stimulation solely the ABVN. FIG. 3C illustrates select anatomy of the ear 390, including a relatively medial area of the ear 390 generally innervated by the auriculotemporal nerve 399, the tragus 398, the helix 397, the concha 396, an area innervated by the great auricular nerve 395 generally at the inferior and lateral edge of the ear, and an area innervated by the ABVN 394 more centrally and generally in the vicinity of the cymba concha or tragus 398. In some embodiments, any number of the aforementioned anatomical locations of the ear can be neuromodulated or configured to be neuromodulated. In some embodiments, any number of the aforementioned anatomical locations of the ear are not neuromodulated or not configured to be neuromodulated.

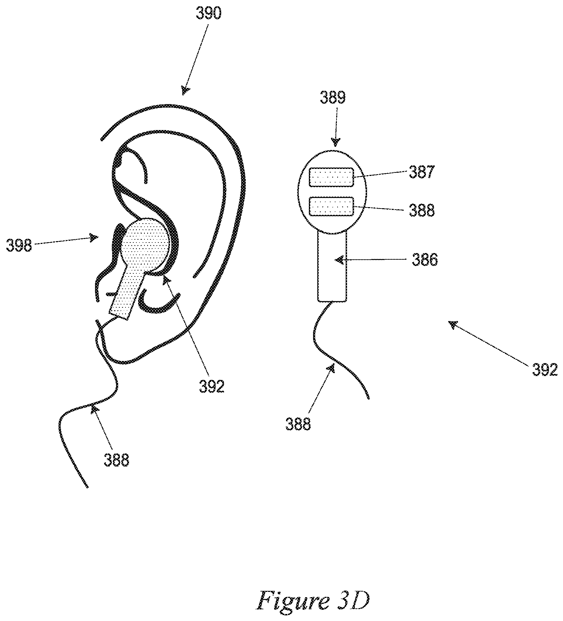

Stimulation of the cymba concha or tragus can occur, for example, noninvasively via a plug, earpiece, or other device that can include electrodes for transcutaneous electrical stimulation in some cases. FIG. 3D illustrates an embodiment of a tragus stimulator 392 with an earbud configuration positioned in the tragus 398 of the ear 390. The stimulator 392 can be wired as shown, or wireless in other embodiments. The stimulator 392 can include a distal ear receptacle portion 389 that can include a cathode 387 and an anode 388, a hub 386 proximate the receptacle portion 389, and a conduit 388 to a source of electromagnetic energy, such as electrical energy. In some embodiments, the auricular stimulator 392 includes one or more sensors for measuring parameters relating to stimulation and/or physiologic function as discussed elsewhere herein. The auricular stimulator 392 can be unilateral or bilateral (e.g., placed in both ears).

In some embodiments, a system can include a plurality of stimulators that communicate with each other wirelessly and provided a synchronized, patterned stimulation. In some embodiments, multiple stimulators may be in electrical connection with multiple electrode pairs to stimulate multiple nerves simultaneously. In one embodiment, a system can include a stimulator on the wrist to target median nerve and a stimulator in the ear to target the ABVN. Each stimulator in the system can communicate with each other via a wired or wireless connection. Multiple stimulators can provide synchronized stimulation to the multiple nerves. Stimulation may be, for example, burst, offset, or alternating between the multiple nerves.

The device could also be responsive to number of episodes of symptoms, including unilateral throbbing cranial pain, sensory sensitivity to light, sound, and smell, nausea, and dysfunction of autonomic, cognitive, emotional, and motor systems in some cases. If more episodes occur in one day, treatment can be increased by increasing the amplitude of the stimulation, duration of the stimulation, or number of treatment sessions, for example.

The number of episodes of symptoms could be detected in various ways to control the stimulation applied by system and devices. In some embodiments, the patient can enter events related to symptoms, including but not limited to unilateral throbbing cranial pain, sensory sensitivity to light, sound, and smell, nausea events on a mobile device.

One embodiment of the system could centrally store biological measures from multiple wearers on a server system (e.g., the cloud), along with other relevant demographic data about each user, including age, weight, height, gender, ethnicity, etc. Data collected from multiple wearers can be analyzed using standard statistical analysis, machine learning, deep learning, or big data techniques, such as a logistic regression or Naive Bayes classifier (or other classifiers), to improve prediction of migraine or headache attacks by determining correlations between biological measures and other recorded symptom events and migraine or headache events. These correlations can be used to set parameters of the stimulation waveform applied by the therapy unit, determine best time to apply stimulation therapy, and/or adapt the stimulation waveform applied by the therapy unit in real time.

In one embodiment of the system, the wearable monitor automatically detects and records the dosage and consumption of medications to (1) track compliance of the patient; (2) combine with the logging of migraine or headache episodes to assess therapeutic effectiveness, and (3) determine or predict migraine or headache attacks. The dosage and consumption of medications can be detected and record in multiple ways, including (1) using visual scanner to record a marking on the pill pack or bottle each time medication is consumed, (2) a smart pill cap with force sensors and a wireless transmitter to detect each time the medication is consumed from a pill bottle, (3) an RFID chip that is of similar size and shape as a pill that is consumed with each dosage of medication that is activated by digestion and communicates with the monitor device, (4) an RFID chip embedded in a sugar pill that is consumed with each dosage of medication that is activated by digestion and communicates with the monitor device, (5) a pill with a visual encoding that is scanned and recorded by a camera on the monitor unit each time medication is consumed, or (6) by having the patient logging drug consumption into the device.

The system can also log the patient satisfaction after each stimulation session or the end of a specified period, like a day or week or month, via an input on the device, which provides another piece of information to help feedback application of therapy. In some cases, if a person is satisfied, the therapy is maintained at the current stimulation waveforms and levels. In other cases, this may mean that the stimulation treatment may need to be optimized, for example, by changing stimulation parameters such as waveform frequency or amplitude.

In some embodiments, the wearable monitor can have a visual, auditory, tactile (e.g., squeezing band), or vibrotactile cues to notify the wearer of key events based on analysis of biological measures, including, but not limited to, prediction of migraine or headache attacks, and/or increase in stress level, heart rate, heart rate variability, or other parameters. The cuing system could also notify the wearer of other predetermined events or reminders set by the wearer.

In some embodiments, the form of the wearable monitor and/or therapy unit could be a wrist band or watch, a ring, a glove, an arm sleeve or arm band or cuff, knee band, sock, leg sleeve or cuff, an ear piece/headphone, head band, a necklace or neck band, or a compliant patch that conforms to multiple locations on the body. In some embodiments, the wearable monitor and/or therapy unit is not in the form of a patch. In some embodiments, the wearable monitor and/or therapy unit extends completely circumferentially around an anatomical location.

In one embodiment, the wearable monitor can have a processing unit and memory that collects, stores, processes, and analyzes the biological measures, along with other data input by the wearer.

In some embodiments, the wearable monitor can take user input about events, including diet history, medication history, caffeine intake, alcohol intake, sodium intake, etc. The monitor can use accelerometers to measure specific movements, gestures, or tapping patterns to record user inputs at specific prompts. Other touch sensors, such as resistive strips or pressure sensitive screens, could be used to measure specific gestures to record user inputs. These gesture-based measures to record user input minimize the complexity of steps required to input user data into the device. The data can be stored in memory and processed by the processing unit. In some embodiments, the data can be transmitted from the wearable monitor to an external computing device.

In one embodiment, the wearable monitor and/or the therapy unit can connect with other applications, such as calendars and activity logs, to sync and track events or a saved calendar can be saved and stored on the device. In some embodiments, the wearable monitor and/or the therapy unit can communicate with a variety of computing devices, such as a smart phone, a smart watch, a tablet, a laptop computer, or a desktop computer, for example, that have these applications. In some embodiments, the wearable monitor can include an ambulatory blood pressure monitor.

In one embodiment, the monitor unit and/or therapy unit can have a GPS or similar device to track the location and assess activity of the wearer. GPS measures can be combined with mapping or location systems to determine context of the wearer's activity (e.g., gym, office, home) or determine changes in elevation during specific activities, such as running or cycling.

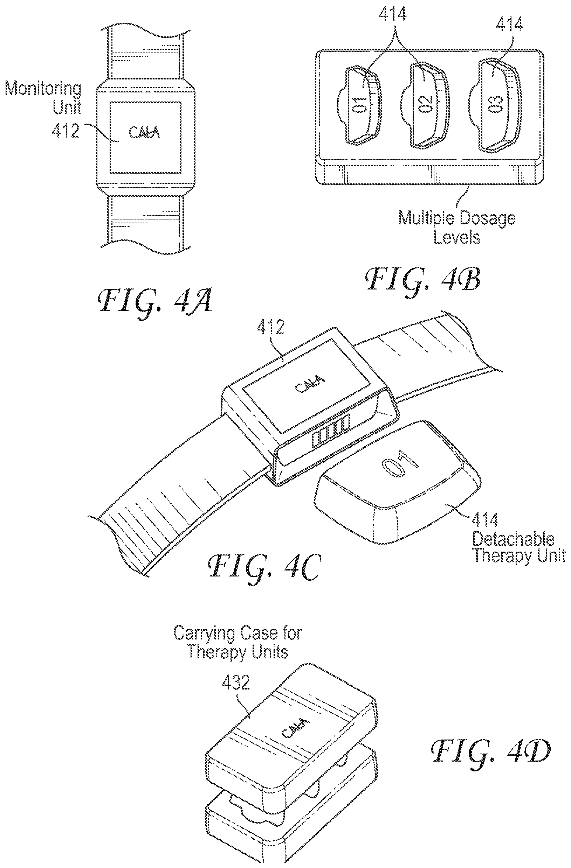

In some embodiments as shown in FIGS. 4A-4D, a single monitor unit 412 can be used with a plurality of therapy units 414 having different sizes, shapes, colors, markings and/or capabilities, which includes different battery capacity and power output. Different wearers and usage scenarios may require different amounts of stimulation duration and power, making a smaller or larger therapy unit more desirable and giving the wearer options to meet their needs in different scenarios. In some embodiments, the therapy units 412 can also have different programming, including different stimulation parameters and/or therapies which can be tailored to different types of treatments. In some embodiments, the therapy units can each be tailored to provide different intensity of treatments, such as one unit for short, low intensity duration treatment and another for long, high intensity treatment, or for various usage patterns or dosing regimens, such as one unit for daily stimulation and one unit for weekly stimulation. The different features and capabilities of the therapy units can correspond to the different sizes, shapes, color, and/or markings. A carrying case 432 can be used to hold a set of therapy units, such as a set of therapy units that differ in battery capacity and power output or some other feature.

In one embodiment, the therapy units have a unique charging station that can simultaneously charge multiple therapy units. The charging station could have a custom direct electrical connection to the therapy units or could charge the therapy units wirelessly in a close proximity. Similarly, in some embodiments, the charging station can charge the monitoring units in a similar manner.

In one embodiment, the wearable monitor can track parameters about stimulation provided by the therapy unit, including time of stimulation, duration of the stimulation session, and power used by the therapy unit. This data can be stored on memory in the wearable monitor, processed by the wearable monitor, and/or transmitted to an external computing device.

In one embodiment, the therapy unit can use switches or an electrical sensor to detect connection of electrodes: (1) to ensure proper and unique electrodes are being installed (i.e., not using a different or incorrect type of electrode) communicating a unique code, for example via RFID, an encoded EEPROM chip, a resistance or capacitance based ID, a binary identifier, or a surface pattern (2) to regulate the number of uses for each electrode or lifetime of the electrode to prevent over use, and (3) to prevent the usage of the device without an electrode to prevent small shock. In some embodiments, the therapy unit and/or the monitor unit can have an identifier that can be transmitted to and be received by each other or to an external computing device. The identifier can allow one unit to determine the features, capabilities, and/or configuration of the other device, including the electrode configuration described above, so that the appropriate treatment parameters can be used, and also the usage life or expiration of the component, which can be based on voltage measurements, time, number of therapy sessions, or other parameters. In some embodiments, instead of using an identifier, the features, capabilities, and/or configuration of one device can be transmitted to the other device, either directly from one device to the other device, or through entry into the user interface, or through an external computing device.

Other components of the therapy system, including the band, the therapy unit, the monitoring unit, the skin interface, can each have one or more identifiers that performs the functions described above. These identifiers can encode a variety of information as described herein, as well as predetermined dosing regimens, initialization routines, calibration routines, or specific parameters. The identifiers may be associated with a lookup table that stores the encoded information.

In some embodiments, the wearable monitor and/or the therapy unit can communicate with an external computer or device (e.g., tablet, smartphone, smartwatch, or custom base station that includes a charger and communications connection) to store data. Communication between the monitor and external device can be a direct, physical connection, or with a wireless communication connection such as Bluetooth or GSM or cellular.

In one embodiment of the device, the therapy unit has an array of electrodes and one or more sensors, such as pressure sensors, between the therapy unit and the wearer's wrist to measure contact pressure of the skin interface at and/or around the electrodes. Consistent pressure of the skin interface is especially critical for comfort of dry electrode materials. This pressure data can be analyzed to determine which electrodes in the array stimulate the appropriate nerves or to detect changes in skin contact due to motion or other conditions and switch stimulation of the electrode array to an optimal location. These methods are used to (1) assess poor contact of electrodes, and (2) adjust amplitude of stimulation based on pressure measurement.

Increasing contact pressure between the device and the wearer's skin and/or stimulating with electrodes with an adequate contact pressure or above a contact pressure threshold could: (1) increase the surface area of contact, which reduces discomfort, (2) activate deep somatic pain peripheral nerve fibers, which could reduce discomfort from stimulation, which activates superficial pain fibers, (3) reduce the stimulation amplitude needed because it improves stimulation of the targeted nerve (e.g., the electrode is physically closer to the nerve by compression of the surrounding tissue), or (4) reduce the effect of skin motion.

In some embodiments, specific fiber types within a nerve or nerves can be selectively activated (e.g., create action potentials in such specific fiber types) to restore autonomic balance by specifically modulating sympathetic and parasympathetic limbs of the autonomic nervous system (e.g., selectively only one, or more than one of A-alpha, A-beta, A-delta, B, and/or C fibers). In some embodiments, systems and methods do not stimulate or substantially stimulate A-alpha, A-beta, A-delta, B fibers, or C fibers.

In some embodiments, disclosed herein are wearable systems and methods that can utilize transcutaneous sensory stimulation in the form of a burst pattern, e.g., a theta burst pattern to treat migraine or headache conditions, and/or a variety of other conditions, including but not limited to those disclosed herein. Noninvasive peripheral nerve theta burst stimulation may be effective in some cases in driving cortical or spinal plasticity more efficiently than continuous stimulation to reduce symptoms and improve an individual's quality of life.

In some embodiments, the stimulation involves patterns of electromagnetic stimulation of peripheral nerves. The patterned stimulation could be a bursting stimulation, such as an on/off pattern that repeats at regular intervals (e.g., on for 10ms, off for 20ms, etc.), or non-burst patterned stimulation that can be more complex in some embodiments, such as a stochastic pattern or a sinusoidal envelope for example. The electromagnetic stimulation could include, for example, electrical energy, mechanical energy (e.g., vibration), magnetic energy, ultrasound energy, radiofrequency energy, thermal energy, light energy (such as infrared or ultraviolet energy for example), and/or microwave energy, or combinations thereof. In some embodiments, the stimulation is limited to only electrical energy (e.g., no magnetic or other types of energy are applied). The peripheral stimulation could include transcutaneous, percutaneous, and/or implanted stimulation.

In some embodiments, the stimulation involves non-invasive transcutaneous electrical patterned or burst stimulation of peripheral nerves, including afferent and/or efferent nerves. Not to be limited by theory, but burst stimulation of peripheral nerves can unexpectedly result in one or more of the following compared with conventional or continuous stimulation: greater efficacy; greater plasticity; increased tolerance or tolerability; reduced effects of habituation; increased comfort; and/or reduced treatment time required to achieve the same beneficial effects. Burst stimulation of peripheral nerves, including afferent nerves, can in some cases deliver a more efficacious therapy by remotely accelerating plasticity of one or more central nervous system (e.g., brain and/or spinal cord) circuits, in other words creating plasticity in neural circuits for a period of time that is far longer than the duration of the stimulation session, such as, for example, about or at least about 6 hours, 12 hours, 24 hours, 2 days, 3 days, 4 days, 5 days, 6 days, 7 days, 2 weeks, 3 weeks, 1 month, 2 months, 3 months, 4 months, 5 months, 6 months, 9 months, 12 months, 18 months, 24 months, 36 months, or even longer. Peripheral stimulation in some cases can be more convenient and comfortable for the user than central stimulation (e.g., transcranial stimulation and/or spinal stimulation) and can be more suitable for home and ambulatory use.

In some embodiments, the burst stimulation includes theta burst stimulation. Theta burst stimulation (TBS) is a patterned form of repetitive stimulation that uses high frequency pulses separated by varying inter-burst intervals. Originally used for the induction of long-term potentiation in hippocampal learning and memory research, theta burst stimulation in the form of repetitive magnetic stimulation (rTMS) has been demonstrated to noninvasively induce plasticity in humans in the motor, sensory and visual cortex. Depending on various parameters including the duration and continuity of stimulation, a long term potentiation or depression (LTP/LTD) like effect can be observed, which are surrogate measures of synaptic efficacy. The number of sessions and the spacing interval between individual sessions of stimulation can also have an effect on the duration of the induced response. The level of muscle relaxation before or during stimulation can also affect the resulting direction or amplitude of plasticity induction suggesting that homeostatic mechanisms are in place that adjust the threshold for plasticity depending on prior synaptic activity. The effective modulation of nervous system plasticity demonstrated with theta burst stimulation can have great potential for the treatment of various neurologic disorders, and can have an effect on other central neural circuits.

In some embodiments, the intraburst frequency could be about or at least about 10 Hz, 20Hz, 30 Hz, 40 Hz, 50 Hz, 100 Hz, 250 Hz, 500 Hz, 1 kHz, or more, or ranges including any two of the foregoing frequencies. In some embodiments, intraburst frequency could vary between about 10 Hz and about 20 kHz. Intraburst frequency can also be varied in a random or pseudorandom fashion during the burst to reduce habituation and/or increase comfort. In other embodiments, the intraburst frequency can be between about 10 Hz and about 250 Hz, between about 50 Hz and about 150 Hz, between about 10 Hz and about 100 Hz, between about 100 Hz and about 150 Hz, between about 50 Hz and about 250 Hz, less than about 100 Hz, 95 Hz, 90 Hz, 85 Hz, 80 Hz, 75 Hz, 70 Hz, 65 Hz, 60 Hz, 55 Hz, 55, Hz, or between about 50 Hz to about 1000 Hz, or ranges including any two of the frequencies disclosed in this paragraph, in order to maximize therapeutic response, improve comfort, reduce habituation, and/or reduce power consumption of the electrical stimulator device.

In some embodiments, a bursting stimulation is applied to one or more nerves and the interburst frequency can be between about 1 Hz to about 20 Hz, such as between about 4 Hz (250ms between the start of each burst) and about 12 Hz (83 ms), such as between about 4 Hz (250 ms) and about 8 Hz (142 ms) which is generally accepted as the theta band frequency, including about 5 Hz (200 ms), or in some embodiments between about 3.5 Hz and about 7.5 Hz, or between about 6 Hz and about 10 Hz.

In some embodiments, the inter-session frequency can be between about 1 minute and about 12 hours, such as between about 5 minutes and about 120 minutes, between about 5 minutes and about 60 minutes, between about 10 minutes and about 30 minutes, about 5, 10, 15, 20, 25, 30, 35, 40, 45, 50, 55, 60, 75, 90, 120, 180, 240, 300, 360, 420, 480, 540, 600, 660, or 720 minutes, or ranges incorporating any two of the aforementioned values.

In some embodiments, a repetitive patterned stimulation known as quadripulse stimulation could be used, which includes four pulses at a short interval frequency (interstimulus interval of 1.5 ms) repeated at about 0.2 Hz for a period of time, such as about 30 minutes. Quadripulse stimulation has been shown to induce prolonged plasticity. Variation of the intraburst frequency using this paradigm can influence the direction of induced plasticity. These repetitive small pulses could be anywhere between 2-10 pulses or more.

Other burst patterns other than theta burst stimulation can also be used, instead or in addition. Some non-limiting examples include delta (0-4 Hz), alpha (8-12 Hz), beta (12-30 Hz), and gamma (30-100 Hz) inter-burst frequencies. In some embodiments, neuromodulation (e.g., stimulation) with bursting at alpha frequencies can advantageously increase oscillatory frequency. In some embodiments, neuromodulation (e.g., stimulation) with bursting at theta frequencies can advantageously disrupt low frequency oscillations, which can be associated with migraine pathology. In some embodiments, neuromodulation (e.g., stimulation) with bursting at beta or gamma frequencies can disrupt increases in surrounding beta/gamma activity.

In some embodiments, an initial calibration and/or periodic or continuous closed-loop adjustment of bursting stimulation frequency can be performed, for example, based on EEG assessment prior to, during, and/or after therapy (e.g., with either in-office assessment or at home assessment, and/or EEG monitoring during resting state ictal or interictal periods to calculate or otherwise determine the dominant oscillatory frequency). Closed-loop adjustments can be made on timescales of seconds, minutes, hours, days, weeks, or months. A controller can be configured to vary the bursting frequency between two or more frequencies; ramp, random, Gaussian, and others as disclosed, for example, elsewhere herein.

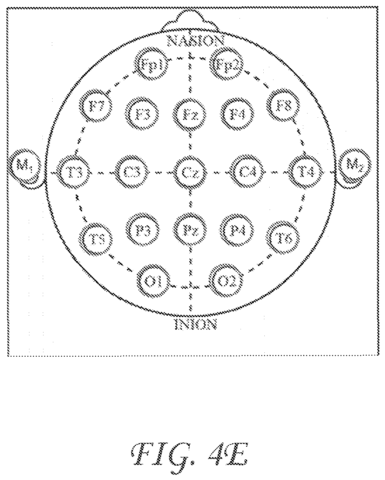

In some embodiments, prior to stimulation, a dominant frequency of aberrant neuron oscillations is calculated from EEG data recorded during a resting state (e.g., interictal state). The EEG data can be recorded from one or more predetermined regions of interest, including but not limited to Fp1, Fp2, F3, F4, F7, F8, C3, C4, T3, T4, T5, T6, P3, P4, M1, M2, O1, and O2, as shown schematically in FIG. 4E. The dominant frequency is then used as a stimulation parameter, such as, for example, limited pattern or burst frequency, stimulation frequency, and/or pulse width. In some embodiments, the dominant frequency of aberrant neuron oscillations can be calculated from EEG data collected over 1, 2, 3, 4, 5, 10, 15, 20, 25, 30, 60, or 90 minutes. The embodiments, the dominant frequency of aberrant neuron oscillations can be re-calculated every 1, 2, 5, 7, 14, 30, 31, 60, or 90 days; 6, 9, or 12 or more months, or ranges incorporating any two of the foregoing values.

In some embodiments, the response to therapy can be observed with EEG assessment after a specified amount of time (e.g., about or no more than about 1, 2, 3, 4, 5, 6, or 7 days; 2, 3, 4 weeks, 1, 2, 3, 4, 5, 6, 7, 8, 9, 10, 11, 12, or more months, or ranges incorporating any two of the foregoing values. For example, a first initial EEG assessment can be performed prior to initial therapy. A second EEG assessment can be performed during or following a therapy session, and the stimulation parameters adjusted based at least in part of the second EEG assessment. In some embodiments, the second EEG assessment can be continuous over a period of time during or following a therapy session, and the stimulation parameters adjusted in real time based at least in part of the EEG data received from the second EEG assessment.

In some embodiments, peripheral burst stimulation can include a sinusoidal, square, rectangular, triangular, sawtooth, or other waveforms.

In some embodiments, burst transcutaneous peripheral electrical stimulation can be preferred in some cases over burst transcutaneous peripheral magnetic stimulation. In some cases, transcutaneous peripheral electrical stimulation can be advantageous because magnetic theta burst can require more power and/or be a heavier device. Electrical stimulation can advantageously provide ambulatory home use, and a more precise stimulation of targeted nerves by controlling flow of current between electrodes or by using a percutaneous needle. In some embodiments, stimulation can be provided at a fixed bursting frequency without measuring for/adjusting for a measured frequency of a physiologic or pathologic parameter or symptom associated with a subject.

In one embodiment, the timing of individual sessions of stimulation can be varied in order to prolong the duration of plasticity. The intersession interval could be between a lower threshold of approximately 1 minute and an upper threshold of approximately 24 hours. Theta burst stimulation intersession interval variation can have a significant effect of varying the spacing intervals between stimulation sessions. Prolongation of the duration of symptom improvement may improve the tolerability of chronic repetitive stimulation. In some embodiments, the intersession interval can be randomized between a lower threshold and an upper threshold. In some embodiments, the intersession interval can increase from a lower threshold or value to an upper threshold or value. In some embodiments, the intersession interval can decrease from an upper threshold or value to a lower threshold or value. In some embodiments, the intersession interval can be varied according to a predetermined algorithm or schedule. In some embodiments, the intersession interval can be varied based on feedback based on data from an accelerometer or electromyography. In some embodiments, the intersession interval can be varied based upon feedback based on tracking symptoms and/or measures of autonomic activity (e.g., HRV, EDA). The interval could also be optimized using machine learning algorithms, such as deep learning, naive Bayesian networks, neural networks, and/or crowdsourced or otherwise aggregated datasets from multiple users with data (e.g., device usage, symptom tracking, autonomic activity) stored on a remote centralized server (e.g., the cloud).

In some embodiments, alternating stimulation of nerves in the wrist (e.g., radial, median, and/or ulnar nerve) or ear can be performed in a rhythmic pattern or pseudorandom pattern. Not to be limited by theory, bursting at a rhythmic pattern can improve efficiency of therapeutic benefit by promoting plasticity of corticospinal circuits. Rhythmic or pseudorandom bursting patterns can prevent habituation of nerves, which occurs with constant stimulation. In some embodiments, rhythmic bursting patterns can be synchronized to heart rhythm events detected by heart rate monitors in the system, including but not limited to an electrical phase of the cardiac cycle, such as the P wave, R wave, QRS complex, ST segment, T wave, and the like.

In some embodiments, median, radial, and/or ulnar stimulation can be combined for a synergistic effect at the brachial plexus. The median, radial, and ulnar nerves innervate different levels of the spinal cord at the brachial plexus, with pathways that proceed to different target locations and organs. Some embodiments can provide timed stimulation, either simultaneously or with a delay, to the median, radial, and/or ulnar nerves to control targeting within the brachial plexus to provide a synergistic effect of neural activation at the brachial plexus, which leads to the stellate ganglia and the sympathetic chain. This synergistic effect can provide an advantage of greater therapeutic benefit with less discomfort and less current (e.g., less power for longer battery life). Timing of the stimulation may be simultaneous, or with a delay to account for differences in conduction velocities for the different nerves such that the signals reach the brachial plexus at the same time. Not to be limited by theory, but simultaneous or near simultaneous activation of the brachial plexus can enhance stimulation through neural pathways. For example, the average conduction velocities of sensory nerves of radial, median, and ulnar nerves are about 51 m/s, 60 m/s, and 63 m/s respectively. Based on variation in nerve length from the wrist to the brachial plexus from 1st percentile female to 99th percentile male, this would require a delay in stimulation between the median and radial nerves of about 1.3 to about 1.7 milliseconds, between median and ulnar of about 0.3 and about 0.4 ms, and between radial and ulnar of about 1.6 ms and about 2.1 ms. In some embodiments the delay in stimulation between a first nerve and a second nerve can be between about 0.3 ms and about 1.7ms, or between about 0.2ms and about 2.0ms, between about 1.2ms and about 2.1ms, or between about 1ms and about 2ms. Lower threshold stimulation on the median, radial, and/or ulnar nerves in combination can advantageously require lower threshold stimulation on the individual nerves with a resultant synergistic effect at the brachial plexus. In some embodiments, a system could include a nerve conduction velocity measurement by applying a stimulation source on a distal portion of the nerve(s) and a measurement electrode on a proximal portion of the nerve(s) to measure an individual's nerve conduction velocities and modify the timed delay based on the individualized measurements.

In some embodiments, a system could include an electrode configuration to stimulate nerves (e.g., radial, median, and/or ulnar) in an alternating pattern that could be rhythmic or pseudorandom. For rhythmic alternating patterns, the alternating frequency can be in a range from 1-100 Hz, which has been shown improve efficiency of therapy by promoting plasticity of corticospinal circuits. In some embodiments, a device embodiment could include an electrode configuration to alternate stimulation of nerves (e.g., radial, median, and/or ulnar) and adjust stimulation parameters (e.g., stimulation frequency, alternating frequency, duration of stimulation, stimulation time of day) based on an assessment of autonomic balance, for example, by measuring heart rate variability (HRV) and analyzing sympathovagal balance as a the ratio of absolute low frequency (LF) to absolute high frequency (HF) power, or LF/HF of measured HRV as noted elsewhere herein.

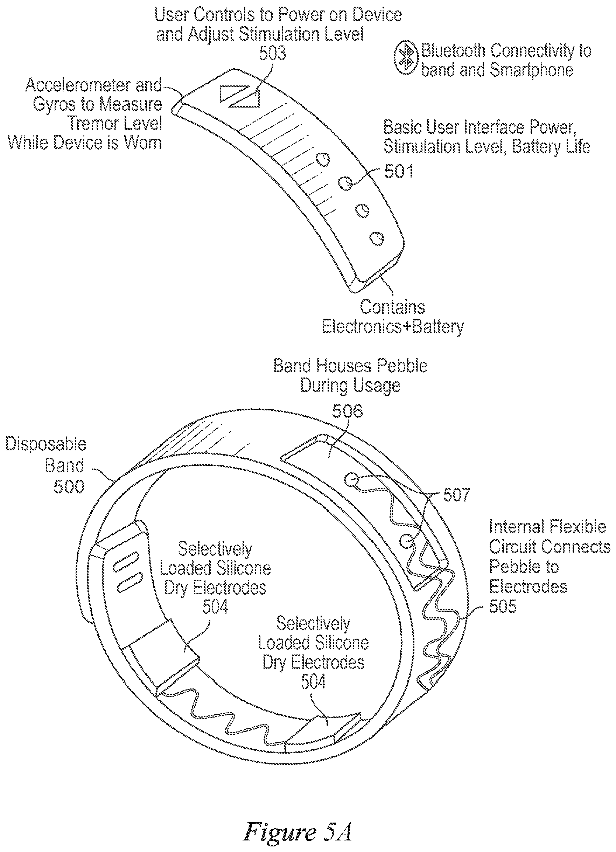

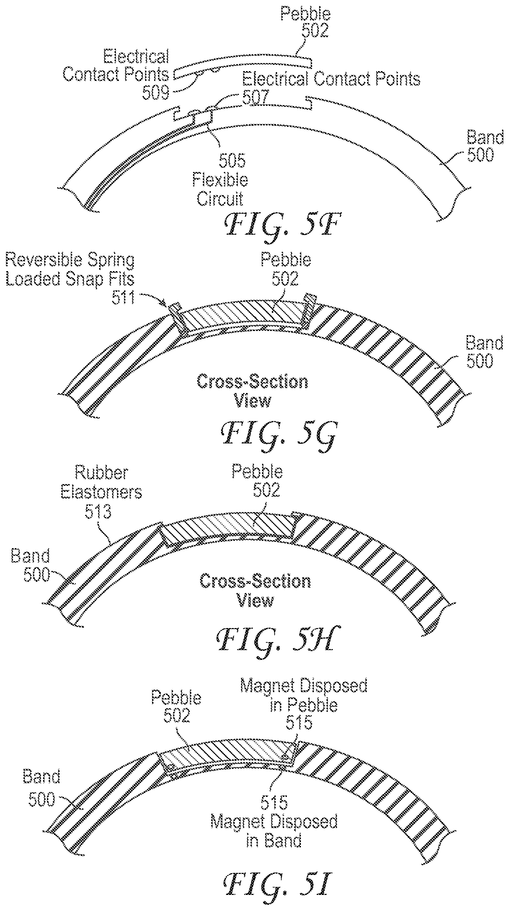

FIGS. 5A-5I illustrates another embodiment of a two-part therapy system that includes a disposable band 500 and a therapy unit 502 that can be reversibly attached to the disposable band 500. The disposable band 500 can have two or more electrodes 504 disposed on a skin facing or inside surface of the band and a receptacle 506 or receiving portion for reversibly receiving the therapy unit 502. Within the band 500 are wires and/or conductive traces that form a flexible circuit 505 that runs from the electrodes 504 to the receptacle 506 for electrically connecting the electrodes 504 to the therapy unit 502 when the therapy unit 502 is disposed in the receptacle 506. In some embodiments, the wires and/or conductive traces of the flexible circuit 505 are arranged in a wave or undulating pattern in order to improve its ability to flex. In some embodiments as shown in FIG. 5F, the receptacle 506 can have one or more electrical contact points, such as one or more pin holes 507, for receiving one or more complementary electrical contacts, such as pins 509, from the therapy unit 502. The flexible circuit 505 can extend to the pin holes 507 such that an electrical connection is formed when the pins are inserted into the pin holes. In some embodiments, as shown in FIGS. 5G-5I, the receptacle 506 can have a clip, retaining lip, magnet, a snap fit, a twist fit, a hook, a latch, a sliding mechanism, or other securement feature for reversibly securing the therapy unit 502 to the band 500. FIG. 5G illustrates clips 511 that may or may not be spring loaded to form a snap fit around the therapy unit 502. FIG. 5H illustrates a flexible lip 513 around the opening of the receptacle that can be used to retain the therapy unit 502 after it is inserted into the receptacle 506. FIG. 5I illustrates magnets 515 that can be placed in complementary positions in the therapy unit 502 and the receptacle. In some embodiments, the clip, magnet, snap fit mechanism, twist fit mechanism, hook, or other securement feature is made of metal or some other conductive material and can be electrically connected to the electrodes via the wires and/or conductive traces. The electrodes 504 can be dry electrodes or can be coated with a conductive gel.

In some embodiments, the therapy unit 502 can include a battery, which may be rechargeable, and electronics to deliver electrical stimulation through the electrodes to the patient's nerves. The electronics can include a stimulator and a microcontroller, and may also include memory and one or more sensors, such as a blood pressure sensor and/or a sensor to measure heart rate and/or heart rate variability and/or galvanic skin response, or one, two, or more ECG electrodes to measure dyssynchrony. In some embodiments, the device is able to sense the impedance of the electrodes in order to assess the integrity of the electrode to skin interface. In some embodiments, there can be an electrical indication (e.g. reading of a chip, pushing in of a sensor on the connector, etc.) to detect integrity of the connection between the band and the therapy unit. In some embodiments, the therapy unit 502 can have one or more LEDs, mini OLED screens, LCS, or indicators 501 that can indicate the status of the therapy unit 502, such as whether the therapy unit 502 is connected to the band 500, the power remaining in the battery of the therapy unit 502, whether a stimulation is being delivered, the stimulation level, whether data is being transmitted, whether a sensor measurement is being taken, whether a calibration routine is being performed, whether the therapy unit 502 is initializing, whether the therapy unit 502 is paired with another device such as a smart watch and/or smart phone, whether the battery is being charged, and the like. In some embodiments, the therapy unit 502 may also include a user interface 503, such as one or more buttons.

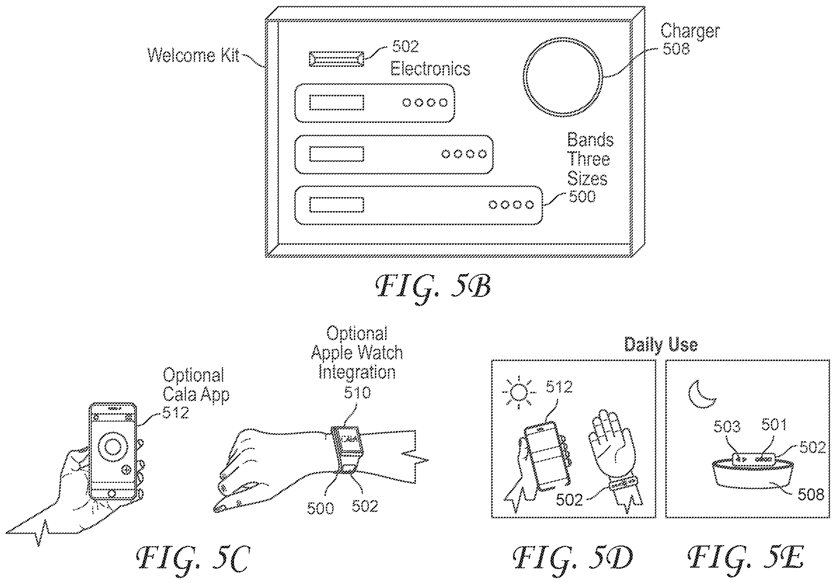

FIG. 5B illustrates a kit including a wrist worn device that can be sent to a user. The kit can contain a plurality of bands 500 of different sizes, shapes, colors, etc. to accommodate patients having different wrist sizes or other body part sizes, such as ankles, arms, fingers, and legs and to accommodate different types of connected accessories like secondary displays (e.g. smart watch). In some embodiments, the kit has three bands to accommodate a majority of wrist sizes. In some embodiments, the kit has two bands to cover most sizes. Additionally, the kit can contain one or more electronic units 502. If multiple electronic units 502 are provided in the kit, the battery capacity of the different electronic units 502 can be different to accommodate different usage types. For example, a relatively low capacity battery can be used for on-demand stimulation, while a relatively high capacity battery can be used for automated and/or responsive stimulation driven by the microcontroller. In some embodiments, only a single electronic unit is provided. In other embodiments, a plurality of electronic units are provided while a single band is provided. The kit may also include a charger 508 to charge the therapy unit 502. In some embodiments, the charger 508 can inductively charge the therapy unit 502. In other embodiments, the charger 508 can charge the therapy unit with a charge cable that can be inserted into a power port in the therapy unit. In some embodiments, the therapy unit 502 can be docked with the charger 508 for charging.

FIG. 5C illustrates an embodiment where a smart watch 510, such as the Apple Watch, is reversibly or permanently fastened to a band 500, which may also have a therapy unit 502. In some embodiments, the smart watch 510 may provide a display and a user interface for the therapy unit 502. The smart watch 510 may communicate with the therapy unit 502 wirelessly, such as through Bluetooth or Wi-Fi, or through a direct connection through a data port in the smart watch and a data port in the therapy unit 502. In some embodiments, the electronic unit 502 and/or smart watch 510 may communicate with a smart phone 512, as described herein, to transmit data or to update the software and/or stimulation parameters on the therapy unit 502 and/or smart watch 510. In some embodiments, the band 500 and therapy unit 502 are permanently affixed or integrated together while the smart watch 510 is reversibly attachable to the band 500. The smart phone 512 and/or the smart watch 510 can include an application, which may be downloaded through the cloud or a computer, configured to interface with the therapy unit 502.

FIGS. 5D and 5E illustrate that the wearable two part system can be worn and used throughout the day. When the power remaining in the battery of the therapy unit is low, the therapy unit 502 can be recharged with the charger 508. Charging can be performed at night or whenever the battery is low or when desired. In some embodiments, the therapy unit can be removed from the band before charging. In some embodiments, the user can swap a low charge therapy unit with a high charged therapy unit so that the user can always be wearing a therapy unit.

In some embodiments, the kit illustrated in FIG. 5B can be used as a diagnostic trial kit. The patient can initially wear the therapy system for about, at least about, or no more than about 1 day to about 90 days, or about or at least about 1, 2, 3, 4, 5, 6, 9, 12, or more months, or for a predetermined length of time. This initial period is used to collect data with the sensors in the therapy unit and/or band in order to characterize the patient's symptomology, or other related measures, or other disease variables, and assess the patient's response to the therapy during the trial period in order to identify how well the patient is responding to the various treatments. The sensor data can be stored in memory in the therapy unit, and/or can be transmitted through a network to the cloud or a server or to another computing device, which can be accessed by the patient's physician, the company, or another third party.

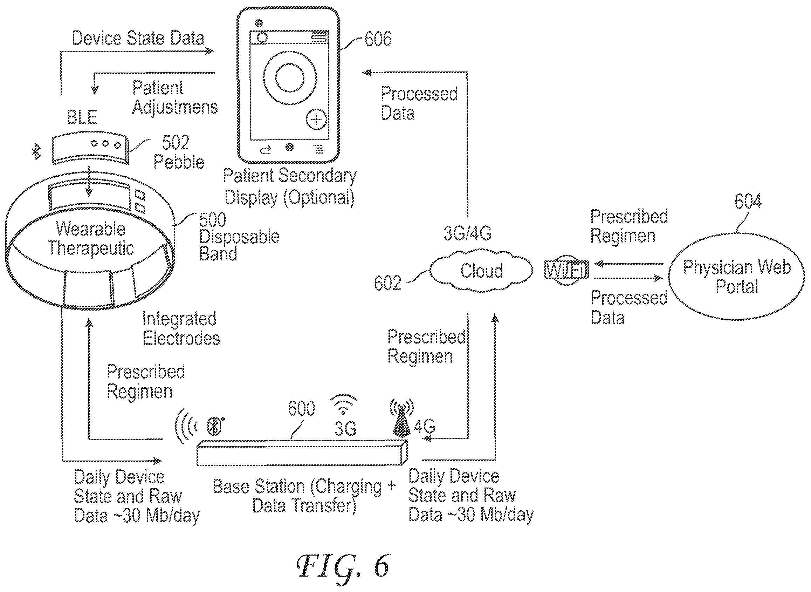

FIG. 6 illustrates an embodiment of a system for treating migraine and optionally other headache conditions using a wearable therapy device. As described above, the therapy device may have two parts, a band 500 and therapy unit 502. A base station 600, which may replace the charger in the kit described above, can be used to both charge the therapy device and to receive and transmit data to the therapy device and to the cloud 602. Communication between the base station 600 and the therapy device can be wireless, such as through Bluetooth and/or Wi-Fi, and communication between the base station 600 and the cloud 602 can be through a cellular network, using a 3G or 4G connection, or through a wired connection to the internet, using DSL or cable or Ethernet, for example. A physician or other user can view and/or retrieve data stored on the cloud 602 using an online portal or a physician web portal 604. In addition, the physician can prescribe and/or modify a treatment regimen on the therapy unit 502 through the cloud 602 and base station 600 using the web portal 604.

In some embodiments, the base station 600 is used to receive and transmit relatively large amounts of data that may require a high bandwidth, such as the transmission of raw data from the therapy device, which may be about 10 to 100 Mb/day, or about 10, 20, 30, 40, or 50 Mb/day. In some embodiments, the data may be stored in memory in the base station 600 and transmitted at another interval, such as weekly or twice weekly, with a scaling up of the bandwidth of transmission. The high bandwidth transmission of the raw data can occur daily while the therapy device is being charged, such as at night during a regular charging period. In some embodiments, the raw data can be processed by the cloud and/or the physician into processed data and sent back to the therapy device.

In some embodiments, the system may optionally include a portable computing device 606, such as a smart phone or tablet, to provide a secondary display and user interface for the patient and to run applications to more easily control the therapy device and view the raw and processed data. The portable computing device can be used to make patient or physician adjustments to the therapy device, such as adjusting the stimulation parameters and dosing, and can receive device state data from the therapy device, which includes data relating to the device, such as when the device was used, errors, therapy parameters such as amplitude and when they were set and delivered. In some embodiments, the portable computing device 606 can receive processed data from the cloud 602 through a cellular network and/or through an internet connection using Wi-Fi, for example.

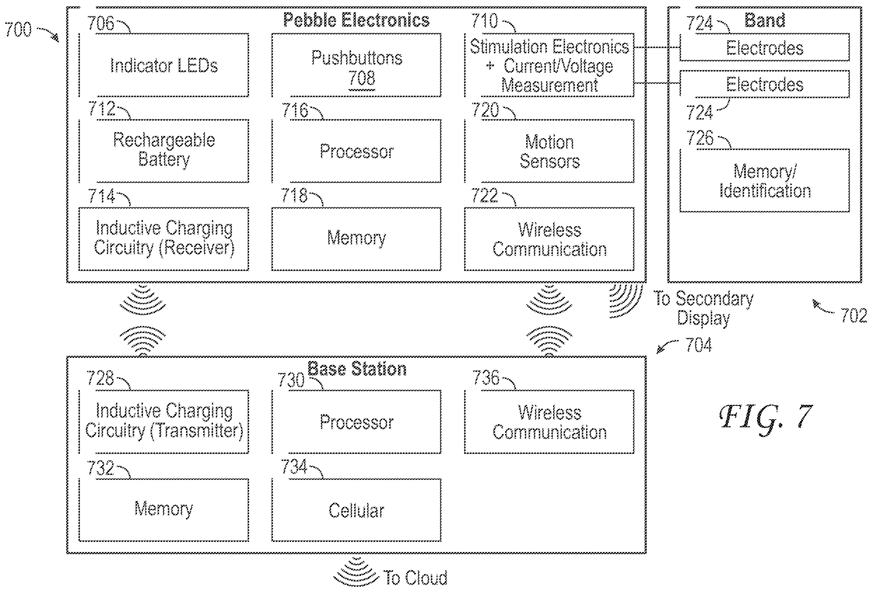

FIG. 7 illustrates the various components that can be included in a therapy unit 700, band 702, and base station 704. These components are described in detail above and also below as one particular embodiment. For example, the therapy unit 700 includes one or more indicators 706, which can be LEDs, and a user interface 708, which can be push buttons, for example. The therapy unit 700 can also have a stimulator 710 with stimulation electronics and may include the capability to measure current and voltage. The therapy unit 700 can also have a battery 712, which may be rechargeable and can be recharged using charging circuitry 714, which may be inductive. The therapy unit 710 may further include a processor 716 and memory 718 to store and execute programs and instructions to accomplish the functions described herein. The therapy unit 710 may also include sensors 720, such as blood pressure sensors, and a communications module 722, which may be wireless and can communicate with the base station 704 and/or a secondary display/computing device.

The band 702 can have electrodes 724 and may also include memory to store identification information or may include some other form of identifier 726 as described herein.

The base station 704 can include charging circuitry 728, which may also be inductive and can transmit power to the complementary charging circuitry 714 on the therapy unit 700. The base station 704 can also have a processor and memory for storing and executing instructions and programs. The base station 704 can further include a communication module 732, which may be cellular, to communicate with the cloud, and another communication module 734, which may be wireless and used to communicate with the therapy unit.

In some embodiments, the device can be a biological sensor, such as a heart rate or respiratory monitor worn on the body, which could include an integrated nerve stimulator. In some embodiments, the nerve stimulator and sensor device can be separate devices that communicate wirelessly. In some embodiments, the device can measure a biological measurement over the course of minutes, hours, days, weeks and/or months to determine whether the patient's condition is increasing, decreasing, or staying the same. In some embodiments, the measurements are time averaged over a window, which can be days, weeks, or months. In some embodiments, a sensor, such as a motion sensor, IMU, or GPS, can be used to detect patient activity, which can affect other measurements. However, some embodiments do not include any motion sensors. In some embodiments, the sensor can be an electrode that measures galvanic skin response, which can be correlated to stress, a known trigger for migraine or headaches. In some embodiments, measurements are collected at the same time each day with the same conditions to improve measurement consistency and to reduce variability. In some embodiments, the stimulator is applied to one wrist or arm or ear to stimulate one peripheral nerve in the arm, such as the median nerve or ABVN, or specific nerve location, such as an acu-pressure point or meridians.

In other embodiments, a stimulator is applied to both wrists/arms or ears to bilaterally stimulate the nerves in the wrist and/or arm, such as median nerves or acu-pressure points, as shown in FIGS. 8A and 8B. In some embodiments, the device can be worn around the wrist, the forearm, or the upper arm, or the leg below the knee, above the knee, or near the ankle or in the ear or on the tragus. In some embodiments, the two bilateral devices can be operated simultaneously to stimulate both nerves at the same time. The stimulation parameters for each device may be the same, or may differ. The two devices may be in communication wirelessly to synchronize or offset the waveforms between to devices. In some embodiments, the two bilateral devices can be operated in an alternating fashion such that only one device delivers stimulation at a time. The alternating devices can alternate stimulation on an hourly, daily, weekly, or monthly basis; and the frequency of the alternation can be modified based on sensor measures.

In some embodiments, the stimulation parameters of the devices described herein are an amplitude of between about 1 mA to about 20 mA, such as between about 1 mA to about 10 mA, or between about 2 mA to about 5 mA. In some embodiments, the frequency can be between about 1 Hz to about 100 kHz, between about 1 Hz and about 150 Hz, or between about 1 Hz and about 10 Hz. In some embodiments, the pulse width can be from about 10 µS to about 1000 µs. In some embodiments, the pulse spacing can be from about 0 µS to about 1000 µs. In some embodiments, the frequency may be a high frequency stimulation, and include frequencies from about 100 Hz to about 100 kHz. In some embodiments, the stimulation waveform is biphasic (i.e., positive portion of a pulse is followed, substantially immediately, by a negative portion of the pulse or vice-versa) or monophasic square wave, sine wave, triangle wave, or other shapes. Other embodiments can include curved waveforms where there can be a ramp-up and/or ramp-down period to or from maximum amplitude. In some embodiments, the stimulation is symmetric or asymmetric. In some embodiments, the asymmetric waveform can be configured to be charge balanced such that the area under the positive-going pulse can be equal to the area under the negative-going pulse. In some embodiments, the leading pulse has a positive polarity or a negative polarity.

In some embodiments, a system is not configured to stimulate the trigeminal nerve (and methods, useful for understanding the invention, do not stimulate the trigeminal nerve). In some embodiments, a system is not configured to be placed on the forehead, other parts of the head, and/or the neck (and methods, useful for understanding the invention, do not involve placement of a stimulation component on the forehead, other parts of the head, and/or the neck). In some embodiments, a system does not include self-adhesive electrodes. In some embodiments, electrodes of the system are not magnetically connected to the stimulation device. In some embodiments, systems deliver only electrical energy, such as only transcutaneous electrical energy, and do not involve any one or more of: magnetic energy (e.g., including transcranial magnetic stimulation (TMS)), ultrasound energy, RF energy, microwave energy, and/or thermal energy). In some embodiments, a system does not include any implanted components. In some embodiments, systems are not configured for placement on the upper arm (and methods, useful for understanding the invention, do not involve placement of a stimulation component on the upper arm). However, some embodiments can include any number of the foregoing features of this paragraph.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps. However, some embodiments can consist or consist essentially of any number of stated elements or steps disclosed herein.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description. The methods disclosed herein include certain actions taken by a practitioner; however, they can also include any third-party instruction of those actions, either expressly or by implication. For example, actions such as "percutaneously stimulating an afferent peripheral nerve" includes "instructing the stimulation of an afferent peripheral nerve."

## Claims

1. A wearable device (312) for treating migraines using transcutaneous peripheral nerve stimulation, comprising:
a first peripheral nerve effector configured to be placed on a skin surface proximate a median nerve of an arm or a wrist of a patient;
a second peripheral nerve effector configured to be placed on a skin surface proximate a nerve other than the median nerve of the arm or the wrist of the patient; and
a controller (321) configured to:
receive data relating to the patient;
generate parameters of a first electrical stimulation signal and/or a second electrical stimulation signal based on the data relating to the patient;
transcutaneously deliver the first electrical stimulation signal to the first peripheral nerve effector to stimulate the median nerve; and
transcutaneously deliver the second electrical stimulation signal to the patient via the second peripheral nerve effector,
wherein the data relating to the patient comprises information related to a number of episodes of symptoms, wherein said symptoms include one or more of: unilateral throbbing cranial pain, sensory sensitivity to light, sound, and smell, nausea, and dysfunction of autonomic, cognitive, emotional, and motor systems;
wherein if more episodes occur in one day, the first and/or second electrical stimulation signal can be increased by increasing an amplitude of the stimulation, a duration of the stimulation, or a number of treatment sessions; and
wherein the controller (321) is configured such that transcutaneously delivering the first electrical stimulation signal and the second electrical stimulation signal is therapeutically effective to modify at least one neural pathway associated with migraine.

2. The device (312) of Claim 1, wherein the controller is configured to adjust the first electrical stimulation signal upon identifying at least one of:
abnormal sympathetic activity in the patient; and
abnormal parasympathetic activity in the patient.

3. The device (312) of any one of the preceding claims,
wherein the data comprises cardiac and/or pulmonary activity of a patient;
wherein the device is configured to compared said data with a reference threshold value algorithmically derived from previously-received data to find a predefined relation; and wherein said first and/or second stimulation signal is delivered when the predefined relation is found.

4. The device (312) of any one of the preceding claims, wherein the device is configured to stimulate the arm only below an elbow.

5. The device (312) of any one of the preceding claims, wherein the first electrical stimulation signal comprises burst stimulation signals.

6. The device (312) of any one of the preceding claims, wherein the device is arranged to deliver the first and second electrical stimulation signals to the patient's wrist, and wherein the device is a non-implantable device that at least partially encircles the wrist.

7. The device (312) of any one of claims 1 to 5 wherein the device is arranged to deliver the first electrical stimulation signal to the patient's wrist and the second electrical stimulation signal to the patient's ear.

8. The device (312) of any one of the preceding claims,
wherein the controller is arranged to receive measurements from a sensor that detects events or phases of a respiratory cycle; and
wherein the controller is configured to activate at least the first electrical stimulation signal or the second electrical stimulation signal in phase with a portion of the respiratory cycle of the patient using measurements from the sensor.

9. The device (312) of any one of the preceding claims, wherein the data is EEG data.

10. The device (312) of any one of claims 1-9, wherein the first electrical stimulation signal has a frequency of 5-150 Hz.

11. The device (312) of any one of claims 1-9, wherein the first electrical stimulation signal has a frequency of between 3 Hz and 15 Hz, and the first electrical stimulation signal is delivered transcutaneously at the wrist.

12. The device (312) of any one of claims 1-6, wherein the device is configured to receive data from a sensor that measures any one of: autonomic nervous system activity of the patient; heart rate variability of the patient; electrodermal activity of the patient; thermometry data of the patient; or ECG activity of the patient.

## Patentansprüche

1. Tragbare Vorrichtung (312) zur Behandlung von Migräne unter Verwendung von transkutaner peripherer Nervenstimulation, umfassend:
einen ersten peripheren Nerveneffektor, der so konfiguriert ist, dass er auf einer Hautoberfläche in der Nähe eines Mediannervs eines Arms oder eines Handgelenks eines Patienten platziert werden kann;
einen zweiten peripheren Nerveneffektor, der so konfiguriert ist, dass er auf einer Hautoberfläche in der Nähe eines anderen Nervs als dem Mediannerv des Arms oder des Handgelenks des Patienten platziert werden kann; und
eine Steuervorrichtung (321), die konfiguriert ist zum:
Empfangen von Daten, die sich auf den Patienten beziehen;
Erzeugen von Parametern eines ersten elektrischen Stimulationssignals und/oder eines zweiten elektrischen Stimulationssignals auf der Grundlage der Daten, die sich auf den Patienten beziehen;
transkutanen Abgeben des ersten elektrischen Stimulationssignals an den ersten peripheren Nerveneffektor, um den Mediannerv zu stimulieren; und
transkutanen Abgeben des zweiten elektrischen Stimulationssignals über den zweiten peripheren Nerveneffektor an den Patienten,
wobei die Daten, die sich auf den Patienten beziehen, Informationen über eine Anzahl von Episoden von Symptomen umfassen, wobei die Symptome eines oder mehrere der folgenden Symptome umfassen: einseitige pochende kraniale Schmerzen, sensorische Empfindlichkeit gegenüber Licht, Geräuschen und Gerüchen, Übelkeit und Funktionsstörungen des autonomen, kognitiven, emotionalen und motorischen Systems;
wobei, wenn mehrere Episoden an einem Tag auftreten, das erste und/oder zweite elektrische Stimulationssignal durch Erhöhen einer Amplitude der Stimulation, einer Dauer der Stimulation oder einer Anzahl von Behandlungssitzungen erhöht werden kann; und
wobei die Steuervorrichtung (321) so konfiguriert ist, dass die transkutane Abgabe des ersten elektrischen Stimulationssignals und des zweiten elektrischen Stimulationssignals therapeutisch wirksam ist, um mindestens eine mit Migräne assoziierte Nervenbahn zu modifizieren.

2. Vorrichtung (312) nach Anspruch 1, wobei die Steuervorrichtung so konfiguriert ist, dass sie das erste elektrische Stimulationssignal anpasst, wenn mindestens eines der folgenden Merkmale festgestellt wird:
eine abnorme sympathische Aktivität beim Patienten; und
eine abnorme parasympathische Aktivität beim Patienten.

3. Vorrichtung (312) nach einem der vorstehenden Ansprüche,
wobei die Daten die Herz- und/oder Lungenaktivität eines Patienten umfassen;
wobei die Vorrichtung so konfiguriert ist, dass sie die Daten mit einem Referenzschwellenwert vergleicht, der algorithmisch aus zuvor empfangenen Daten abgeleitet wurde, um eine vordefinierte Beziehung zu finden; und
wobei das erste und/oder zweite Stimulationssignal abgegeben wird, wenn die vordefinierte Beziehung gefunden wird.

4. Vorrichtung (312) nach einem der vorstehenden Ansprüche, wobei die Vorrichtung so konfiguriert ist, dass sie den Arm nur unterhalb des Ellenbogens stimuliert.

5. Vorrichtung (312) nach einem der vorstehenden Ansprüche, wobei das erste elektrische Stimulationssignal Burst-Stimulationssignale umfasst.

6. Vorrichtung (312) nach einem der vorstehenden Ansprüche, wobei die Vorrichtung so angeordnet ist, dass sie das erste und zweite elektrische Stimulationssignal an das Handgelenk des Patienten abgibt, und wobei die Vorrichtung eine nicht implantierbare Vorrichtung ist, die das Handgelenk zumindest teilweise umgibt.

7. Vorrichtung (312) nach einem der Ansprüche 1 bis 5, wobei die Vorrichtung so angeordnet ist, dass sie das erste elektrische Stimulationssignal an das Handgelenk des Patienten und das zweite elektrische Stimulationssignal an das Ohr des Patienten abgibt.

8. Vorrichtung (312) nach einem der vorstehenden Ansprüche,
wobei die Steuervorrichtung so angeordnet ist, dass sie Messungen von einem Sensor empfängt, der Ereignisse oder Phasen eines Atemzyklus erfasst; und
wobei die Steuervorrichtung so konfiguriert ist, dass sie mindestens das erste elektrische Stimulationssignal oder das zweite elektrische Stimulationssignal phasengleich mit einem Teil des Atemzyklus des Patienten unter Verwendung von Messungen von dem Sensor aktiviert.

9. Vorrichtung (312) nach einem der vorstehenden Ansprüche, wobei die Daten EEG-Daten sind.

10. Vorrichtung (312) nach einem der Ansprüche 1 bis 9, wobei das erste elektrische Stimulationssignal eine Frequenz von 5 bis 150 Hz aufweist.

11. Vorrichtung (312) nach einem der Ansprüche 1 bis 9, wobei das erste elektrische Stimulationssignal eine Frequenz zwischen 3 Hz und 15 Hz aufweist und das erste elektrische Stimulationssignal transkutan am Handgelenk abgegeben wird.

12. Vorrichtung (312) nach einem der Ansprüche 1 bis 6, wobei die Vorrichtung so konfiguriert ist, dass sie Daten von einem Sensor empfängt, der eines der folgenden Merkmale misst: die Aktivität des autonomen Nervensystems des Patienten; die Herzfrequenzvariabilität des Patienten; die elektrodermale Aktivität des Patienten; Thermometriedaten des Patienten; oder die EKG-Aktivität des Patienten.

## Revendications

1. Dispositif portable (312) pour traiter les migraines à l'aide d'une stimulation nerveuse périphérique transcutanée, comprenant :
un premier effecteur de nerf périphérique configuré pour être placé sur une surface de peau à proximité d'un nerf médian d'un bras ou d'un poignet d'un patient ;
un deuxième effecteur de nerf périphérique configuré pour être placé sur une surface de peau à proximité d'un nerf autre que le nerf médian du bras ou du poignet du patient ; et
un dispositif de commande (321) configuré pour :
recevoir des données relatives au patient ;
générer des paramètres d'un premier signal de stimulation électrique et/ou d'un deuxième signal de stimulation électrique sur la base des données relatives au patient ;
administrer par voie transcutanée le premier signal de stimulation électrique au premier effecteur de nerf périphérique pour stimuler le nerf médian ; et
administrer par voie transcutanée le deuxième signal de stimulation électrique au patient par l'intermédiaire du deuxième effecteur de nerf périphérique,
dans lequel les données relatives au patient comprennent des informations liées à un certain nombre d'épisodes de symptômes, dans lequel lesdits symptômes incluent un ou plusieurs parmi : une douleur crânienne pulsatile unilatérale, une sensibilité sensorielle à la lumière, au son, et à l'odeur, des nausées et un dysfonctionnement des systèmes autonome, cognitif, émotionnel et moteur ;
dans lequel, si plus d'épisodes se produisent au cours d'une journée, les premier et/ou deuxième signaux de stimulation électrique peuvent être augmentés par augmentation de l'amplitude de la stimulation, d'une durée de la stimulation, ou d'un nombre de séances de traitement ; et
dans lequel le dispositif de commande (321) est configuré de sorte que l'administration par vois transcutanée du premier signal de stimulation électrique et du deuxième signal de stimulation électrique est thérapeutiquement efficace pour modifier au moins une voie neuronale associée à la migraine.

2. Dispositif (312) selon la revendication 1, dans lequel le dispositif de commande est configuré pour régler le premier signal de stimulation électrique après avoir identifié au moins l'une parmi :
une activité sympathique anormale chez le patient ; et
une activité parasympathique anormale chez le patient.

3. Dispositif (312) selon l'une quelconque des revendications précédentes,
dans lequel les données comprennent l'activité cardiaque et/ou pulmonaire d'un patient ;
dans lequel le dispositif est configuré pour comparer lesdites données à une valeur seuil de référence dérivée algorithmiquement à partir de données reçues précédemment afin de trouver une relation prédéfinie ; et
dans lequel lesdits premier et/ou deuxième signaux de stimulation sont administrés lorsque la relation prédéfinie est trouvée.

4. Dispositif (312) selon l'une quelconque des revendications précédentes, dans lequel le dispositif est configuré pour stimuler le bras uniquement en dessous d'un coude.

5. Dispositif (312) selon l'une quelconque des revendications précédentes, dans lequel le premier signal de stimulation électrique comprend des signaux de stimulation en rafale.

6. Dispositif (312) selon l'une quelconque des revendications précédentes, dans lequel le dispositif est agencé pour administrer les premier et deuxième signaux de stimulation électrique au poignet du patient, et dans lequel le dispositif est un dispositif non implantable qui entoure au moins partiellement le poignet.

7. Dispositif (312) selon l'une quelconque des revendications 1 à 5 dans lequel le dispositif est agencé pour administrer le premier signal de stimulation électrique au poignet du patient et le deuxième signal de stimulation électrique à l'oreille du patient.

8. Dispositif (312) selon l'une quelconque des revendications précédentes,
dans lequel le dispositif de commande est agencé pour recevoir des mesures à partir d'un capteur qui détecte des événements ou des phases d'un cycle respiratoire ; et
dans lequel le dispositif de commande est configuré pour activer au moins le premier signal de stimulation électrique ou le deuxième signal de stimulation électrique en phase avec une portion du cycle respiratoire du patient à l'aide de mesures à partir du capteur.

9. Dispositif (312) selon l'une quelconque des revendications précédentes, dans lequel les données sont des données EEG.

10. Dispositif (312) selon l'une quelconque des revendications 1 à 9, dans lequel le premier signal de stimulation électrique a une fréquence de 5 à 150 Hz.

11. Dispositif (312) selon l'une quelconque des revendications 1 à 9, dans lequel le premier signal de stimulation électrique a une fréquence comprise entre 3 Hz et 15 Hz, et le premier signal de stimulation électrique est administré par voie transcutanée au niveau du poignet.

12. Dispositif (312) selon l'une quelconque des revendications 1 à 6, dans lequel le dispositif est configuré pour recevoir des données à partir d'un capteur qui mesure l'une parmi : une activité du système nerveux autonome du patient ; une variabilité de rythme cardiaque du patient ; une activité électrodermique du patient ; des données thermométriques du patient ; ou une activité ECG du patient.
